(19) Europäisches Patentamt
European Patent Office
Office européen des brevets

(11) EP 3 909 947 A2

(12) EUROPEAN PATENT APPLICATION
published in accordance with Art. 153(4) EPC

(43) Date of publication:
**17.11.2021 Bulletin 2021/46**

(21) Application number: **20756552.4**

(22) Date of filing: **21.02.2020**

(51) Int Cl.:
*C07D 309/38* (2006.01) *C12N 15/70* (2006.01)
*C12N 9/02* (2006.01) *C12P 17/06* (2006.01)

(86) International application number:
**PCT/IB2020/051457**

(87) International publication number:
**WO 2020/165882 (20.08.2020 Gazette 2020/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.02.2019 KR 20190016016**
**21.06.2019 KR 20190074404**
**03.09.2019 KR 20190109179**

(71) Applicant: **Korea Research Institute of Chemical Technology**
**Daejeon 34114 (KR)**

(72) Inventors:
- **CHA, Hyun Gil**
  **Daejeon 34114 (KR)**
- **JOO, Jeong Chan**
  **Daejeon 34114 (KR)**
- **KIM, Hee Taek**
  **Daejeon 34114 (KR)**
- **KIM, Kyung An**
  **Daejeon 34114 (KR)**
- **PARK, Jeyoung**
  **Daejeon 34114 (KR)**
- **KANG, Myung Jong**
  **Daejeon 34114 (KR)**
- **HWANG, Sung Yeon**
  **Daejeon 34114 (KR)**
- **OH, Dong Yeop**
  **Daejeon 34114 (KR)**
- **LEE, Min Woo**
  **Daejeon 34114 (KR)**

(74) Representative: **Witte, Weller & Partner Patentanwälte mbB**
**Postfach 10 54 62**
**70047 Stuttgart (DE)**

# (54) METHOD OF PRODUCING HIGH-PURITY 2-PYRONE-4,6-DICARBOXYLIC ACID AND METHOD OF PRODUCING INTERMEDIATE FOR SAME

(57) The present invention relates to a method of producing high-purity 2-pyrone-4,6-dicarboxylic acid (PDC), and a method of producing an intermediate for the same, the methods comprising the steps of: 1) producing terephthalic acid (TPA) from PET; 2) producing PDC by reacting TPA with a recombinant strain; and 3) purifying the produced PDC to a high purity. By using a biomass-derived $SiO_2$ catalyst, a preparation method of the same, and a disintegration method from PET to TPA using the same according to the present invention, high-purity TPA can be produced at a faster rate than a conventional PET hydrolysis rate under neutral conditions, wherein the produced TPA can be usefully utilized as a raw material for polyester fibers and the like. The present invention relates to a recombinant strain for producing PDC introduced with nucleotide sequences that encode the enzymes TphB, TphAabc, LigAB, and LigC, and to a method of producing PDC, comprising reacting the recombinant strain for producing PDC and a substrate. The present invention provides a method of purifying PDC to a high purity by adding acetone to precipitate and remove impurities which are confirmed by NMR and/or TLC analysis, but hardly removed and purified, from crude 2-pyrone-4,6-dicarboxylic acid or its sodium salt which is industrially manufactured or commercially available, extracting and concentrating the resulted solution with an organic solvent, and eluting the resulted concentrate with a mixed solvent of ethyl acetate and hexane by column chromatography.

EP 3 909 947 A2

[FIG. 1]

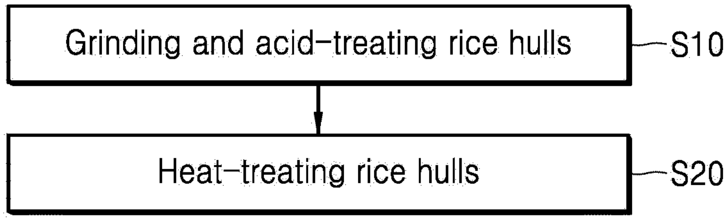
Grinding and acid-treating rice hulls
S10
Heat-treating rice hulls
S20

<110> KOREA RESEARCH INSTITUTE OF CHEMICAL TECHNOLOGY

<120> PURIFICATION OF HIGH PURITY 2-PYRONE-4,6-DICARBOXYLIC ACID AND PREPARING INTERMEDIATE THEREFOR

<130> KPA190798-KR

<150> KR 10/20190074404
<151> 2019-06-21

<160> 6

<170> KoPatentIn 3.0

<210> 1
<211> 960
<212> DNA
<213> Artificial Sequence

<220>
<223> recombinant tphB

<400> 1
```
gaattcatga caatagtgca ccgtagattg gctttggcca tcggcgatcc ccacggtatt        60

ggcccagaaa tcgcactgaa agctctccag cagctgtctg tcaccgaaag gtctcttatc       120

aaggtctatg gaccttggag cgctctcgag caagccgcac gggtttgcga aatggagccg       180

cttcttcaag acatcgttca cgaggaagcc ggcacactta cacaaccagt tcaatgggga       240

gaaatcaccc cgcaggctgg tctatctacg gtgcaatccg caacagcggc tatccgagcg       300

tgcgaaaacg gcgaagtcga tgccgtcatt gcctgccctc accatgaaac ggccattcac       360

cgcgcaggca tagcgttcag cggctaccca tctttgctcg ccaatgttct tggcatgaac       420

gaagaccagg tattcctgat gctggtgggg gctggcctgc gcatagtgca tgtcactttg       480

catgaaagcg tgcgcagcgc attggagcgg ctctctcctc agttggtggt caacgcggcg       540

caggctgccg tgcagacatg caccttactc ggagtgccta aaccaaaagt cgctgtattc       600

gggatcaacc ctcatgcatc tgaaggacag ttgttcggcc tggaggactc ccagatcacc       660

gttcccgctg tcgagacact gcgcaagcgc ggcctagcag tagacggccc catgggagct       720

gacatggttc tggcacagcg caagcacgac ctgtatgtag ccatgctgca cgatcagggc       780

catatcccca tcaagctgct ggcacctaac ggagccagcg cactatctat cggtggcagg       840

gtggtgcttt ccagcgtggg ccatggcagc gccatggaca ttgccggccg tggcgtggct       900

gacgccacgg ccctcctacg cacaatagcc ctactcggag cccaaccggt ctaaggtacc       960

                                                                        960
```

<210> 2
<211> 2804
<212> DNA
<213> Artificial Sequence

<220>
<223> recombinant tphAabc

<400> 2
ggtacctttc acacaggaaa cagaccatga accaccagat ccatatccac gactccgata 60

tcgcgttccc ctgcgcgccc gggcaatccg tactggatgc agctctgcag gccggcatcg 120

agctgcccta ttcctgccgc aaaggtagct gtggcaactg tgcgagtacg ctgctcgacg 180

gaaatattgc ctccttcaat ggcatggccg tgcgaaacga actctgcgcc tcggaacaag 240

tgctgctgtg cggctgcact gcagccagcg atatacgtat ccacccgagc tcctttcgcc 300

gtctcgaccc ggaagcccga aaacgtttta cggccaaggt gtacagcaat acactggcgg 360

cacccgatgt ctcgctgctg cgcctgcgcc tgcctgtggg caagcgcgcc aaatttgaag 420

ccggccaata cctgctgatt cacctcgacg acggggaaag ccgcagctac tctatggcca 480

atccacccca tgagagcgat ggcatcacat tgcatgtcag gcatgtacct ggtggtcgct 540

tcagcactat cgttcagcag ttgaagtctg gtgacacatt ggatatcgaa ctgccattcg 600

gcagcatcgc actgaagcct gatgacgcaa ggcccctgat ttgcgttgcg ggtggcacgg 660

gatttgcgcc cattaaatcc gttcttgatg acttagccaa acgcaaggtt cagcgcgaca 720

tcacgctgat ctgggggcct cgcaacccct cgggcctgta tcttcctagc gccatcgaca 780

agtggcgcaa agtctggcca cagtttcgct acattgcagc catcaccgac ctaggcgata 840

tgcctgcgga tgctcacgca ggtcgggtgg atgacgcgct acgcactcac tttggcaacc 900

tgcacgatca tgtggtgcac tgctgtggct caccagctct ggttcaatca gtgcgcacag 960

ccgcttccga tatgggcctg cttgcacagg acttccacgc ggatgttttt gcgacaggcc 1020

cgactggtca ccactaagga tcctttcaca caggaaacag accatgcaag aatccatcat 1080

ccagtggcat ggggccacta atacgcgcgt gccttttggt atctataccg acacagccaa 1140

tgctgatcag gaacagcagc gcatctatcg cggcgaggtc tggaactact tgtgcctgga 1200

atctgaaatt cccggggccg gtgatttccg cactaccttt gccggtgaaa caccgatagt 1260

tgtcgtacgg gatgccgacc aggaaatcta cgccttcgag aaccgctgcg cgcatcgcgg 1320

cgctctcatc gctctggaga aatcgggccg tacggatagt ttccagtgcg tctatcacgc 1380

ctggagctac aaccgacagg gagatctgac cggcgttgcc ttcgagaaag gtgtcaaggg 1440

ccagggtggc atgccggcct cattctgcaa agaagagcat ggcccgcgca agctccgcgt 1500

ggctgtcttt tgcggtttgg tctttggcag tttttccgag gacgtgccca gcattgagga 1560

ttaccttggc cctgagattt gcgagcgcat agagcgcgtg ctgcacaagc ccgtagaagt 1620

catcggtcgc ttcacgcaaa agctgcctaa caactggaag ctctacttcg agaacgtgaa 1680

ggacagctat cacgccagcc tcctgcatat gttcttcacc accttcgagc tgaatcgcct 1740

ctcacaaaaa ggcggtgtca tcgtcgacga gtcgggtggc caccatgtga gctattccat 1800

gatcgatcgc ggcgccaaag acgactcgta caaggaccag gccatccgct ccgacaacga 1860

gcgttaccgg ctcaaagatc ctagccttct agagggcttt gaggagttcg aggacggcgt 1920

gaccctgcag atcctttctg tgttccctgg ctttgtgctg cagcagattc agaacagcat 1980

cgccgtgcgt cagttgctgc ccaagagcat ctccagctcg gaactcaact ggacctatct 2040

tggctatgca gatgacagtg ccgagcaacg caaggtcaga ctcaaacagg ccaaccttat 2100

cggcccggcc ggattcattt ccatggaaga cggagctgtc ggtggattcg tgcagcgtgg 2160

catcgcaggc gctgccaacc ttgatgcagt catcgagatg ggcggagacc acgaaggctc 2220

tagcgagggc cgcgccacgg aaacctcggt acgcggcttt tggaaggcct accgcaagca 2280

tatgggacag gagatgcaag cataacctgc aggtttcaca caggaaacag accatgatca 2340

atgaaattca aatcgcggcc ttcaatgccg cctacgcgaa gaccatagac agtgatgcaa 2400

tggagcaatg gccaaccttt ttcaccaagg attgccacta ttgcgtcacc aatgtcgaca 2460

accatgatga gggacttgct gccggcattg tctgggcgga ttcgcaggac atgctcaccg 2520

accgaatttc tgcgctgcgc gaagccaata tctacgagcg ccaccgctat cgccatatcc 2580

tgggtctgcc ttcgatccag tcaggcgatg caacacaggc cagcgcttcc actccgttca 2640

tggtgctgcg catcatgcat acaggggaaa cagaggtctt tgccagcggt gagtacctcg 2700

acaaattcac cacgatcgat ggcaagttac gtctgcaaga acgcatcgcg gtttgcgaca 2760

gcacggtgac ggacacgctg atggcattgc cgctataaaa gctt 2804

<210> 3
<211> 1389
<212> DNA
<213> Artificial Sequence

<220>
<223> recombinant ligAB

<400> 3
ggtacctttc acacaggaaa cagaccatga ccgagaagaa agagagaatc gacgttcacg 60

cctatctcgc cgagtttgac gacattcccg gcacccgcgt gttcaccgcc cagcgcgcgc 120

gcaagggcta taatctcaac cagttcgcga tgagcctgat gaaggccgag aaccgcgagc 180

ggttcaaggc cgacgagagc gcctatctgg acgagtggaa cctcacgccc gccgccaagg 240

ccgccgtgct ggcccgcgac tacaatgcga tgatcgacga gggcgggaat gtctatttcc 300

tgtccaagct gttctcgacc gacggcaaga gcttccagtt cgccgccggc tcgatgaccg 360

gcatgactca ggaagaatat gcacagatga tgatcgatgg cggccgttcg cccgcgggtg 420

tgcgctcgat caagggaggc tactaaggat cctttcacac aggaaacaga ccatggcacg 480

cgttaccacg ggcatcacgt ccagccacat tcccgcgctc ggcgcggcca tccagaccgg 540

caccagcgac aatgattact ggggcccggt gttcaagggc taccagccga tccgcgactg 600

gatcaagcag cccggcaaca tgccggacgt cgtgatcctg gtctataacg accatgcctc 660

ggccttcgac atgaacatca tcccgacctt cgcgatcggc tgcgcggaaa cgttcaagcc 720

cgccgacgag ggatggggcc cgcgcccggt gcccgacgtg aagggccatc cggaccttgc 780

ctggcacatc gcccagagcc tgatcctcga cgagttcgac atgaccatca tgaaccagat 840

ggacgtcgat catggctgca ccgtgccgct ctcgatgatc ttcggcgagc ccgaggaatg 900

gccgtgcaag gtcatcccct tcccggtcaa tgtcgtcact tatccgccgc cgtcgggcaa 960

gcgctgcttc gcgctcggtg acagcatccg cgccgcggtc gagagcttcc cggaagacct 1020

caacgtccat gtctggggca ccggcggcat gagccaccag cttcagggcc cgcgcgccgg 1080

cctcatcaac aaggagttcg acctgaactt catcgacaag ctgatcagcg accccgagga 1140

gctgagcaag atgccgcaca tccagtatct gcgcgaaagc ggatcggaag gcatcgagct 1200

ggtcatgtgg ctcatcatgc gcggcgcgct gccggagaag gtgcgggatc tctacacctt 1260

ctatcacatc ccggcctcca acaccgcgct cggcgcgatg atcctgcagc cggaggagac 1320

cgcaggtaca ccgctcgaac cgcgcaaggt gatgagcgga cacagcctgg cccaggccta 1380

acctgcagg 1389

<210> 4
<211> 982
<212> DNA
<213> Artificial Sequence

<220>
<223> recombinant ligC

<400> 4
cctgcaggtt tcacacagga aacagaccat gagaatagct ctcgcaggcg ccggcgcatt 60

cggcgaaaaa catctcgacg gtctcaagaa tatcgacggc gtcgagatcg tctcgatcat 120

cagccgcaag gccgagcagg ccgcggaagt cgccgcgaaa tatggcgcga agcacagcgg 180

caccgatctt tccgaagcgc ttgcccgtga cgatgtcgac gcggtgatcc tctgcacccc 240

cacccagatg catgccgagc aggccattgc ctgcatgaat gccggcaagc atgtccaggt 300

ggaaatcccg ctggcggaca gctgggccga tgccgaagcg gtgatgaaga agagccagga 360

aaccggcctc gtctgcatgg tcggccacac ccgccgcttc aacccgagcc accagtatat 420

ccacaacaag atcgtggccg gcgagctcgc catccagcag atggacgtgc agacctattt 480

cttccgccgc aagaacatga acgccaaggg cgagccgcgg tcctggacgg accatctgct 540

ctggcaccat gccgcgcaca cggtggacct gttcgcttat caggccggca agatcgtgca 600

```
ggccaatgcc gtgcaggggc cgatccatcc cgagctcggc atcgccatgg acatgtcgat     660
ccagctcaag agcgagacgg gcgcgatctg caccctgtcc ctctcgttca acaatgacgg     720
gccgctcggc accttcttcc gctacatctg cgacaacggc acctggatcg cgcgctacga     780
cgatctggtg accggcaagg aagaacccgt ggacgtcagc aaggtcgacg tgtcgatgaa     840
cgggatcgag ctgcaggatc gggaatttat cgcggcgatc cgcgaaggtc gcgagcccaa     900
ttcgtcggtg gcgagagtgc tcgattgcta tcgggtgctg ggcgaactcg aggtccagct     960
cgaaaagcag ggctaaaagc tt                                              982

<210>  5
<211>  30
<212>  DNA
<213>  Artificial Sequence

<220>
<223>  primer

<400>  5
ggatccatga caatagtgca ccgtagattg                                       30

<210>  6
<211>  25
<212>  DNA
<213>  Artificial Sequence

<220>
<223>  primer

<400>  6
actagtttag accggttggg ctccg                                            25
```

## Description

### [Technical Field]

The present invention relates to a method of producing high-purity 2-pyrone-4,6-dicarboxylic acid, and a method of producing an intermediate for the same.

According to the present invention, terephthalic acid (TPA) may be produced from polyethylene terephthalate (PET) using a biomass catalyst and 2-pyrone-4,6-dicarboxylic acid (PDC), and 2-pyrone-4,6-dicarboxylic acid (PDC) may be produced by reacting the TPA with a recombinant strain. Further, high-purity PDC may be provided by removing impurities of the PDC.

Further, the present invention relates to a method of hydrolyzing polyethylene terephthalate to terephthalic acid using a $SiO_2$ catalyst derived from a biomass and microwaves.

More specifically, the present invention relates to a recombinant strain for producing 2-pyrone-4,6-dicarboxylic acid (PDC) introduced with nucleotide sequences that encode enzymes TphB, TphAabc, LigAB, and LigC, and to a method of producing PDC including reacting the recombinant strain for producing PDC with a substrate.

Further, the present invention relates to a method of purifying 2-pyrone-4,6-dicarboxylic acid to a high purity.

### [Background Art]

Rice, a main grain of the Asian area, including Korea, has been produced at about 5 million tons per year as about 4.68 million tons (approximately 0.92 million ha of cultivation area) in 2009 in Korea. Rice hulls are agricultural waste that occur in a threshing process of rice, and generally, occupies about 20% of rice at a weight ratio, and only in Korea, about 0.9 to 1 million tons per year of rice hulls have been generated as a by-product of a milling process.

However, despite various utilization studies of rice hulls since the mid-1900s, in Korea, only the self-utilization of rice hulls has been performed, such as most of rugs livestock facilities of cow, pig, etc., an interior thermal insulation, etc.

The rice hulls are largely divided into organic and inorganic material ingredients, wherein the organic material is about 80% and the inorganic material is about 20%. The organic material consists of vegetable polymers such as cellulose, hemicellulose and lignin, and the inorganic material mostly consists of silica. The silica ingredient contained in the rice hulls has a form protected by a polymer that has a large physical and chemical resistance, such as lignin.

Accordingly, in order to enhance the utilization of the rice hulls, various research and developments have been continuously conducted, such as a study for using rice hulls as a raw material, development of technology of preparing a wood material or a synthetic wood by utilizing the rice hulls as a material, and studies of separating/extracting and using silica present in the rice hulls.

Meanwhile, polyethylene terephthalate (PET) that can be obtained by condensation-polymerizing terephthalic acid and ethylene glycol has good rigidity, electrical properties, weather resistance, and heat resistance, a quite small decrease in tensile strength despite long-term exposure under a high temperature, and good resistance to oil due to crystalline plastic.

As a result, the PET has been used as household goods, toys, electrical insulators, household appliance cases, packaging materials, and especially, due to a light weight and no taste and smell, the PET occupies most of plastic beverage bottles.

However, despite these advantages, the PET is very slowly disintegrated because of large resistance to atmospheric and biological materials, so that a waste PET resin causes environmental problems.

As a method of recycling such a waste PET resin, there are disclosed chemical recycling methods such as glycolysis, hydrolysis, methanolysis, etc.

The glycolysis method is a method which is commercially widely used in a PET depolymerization method by glycol such as ethylene glycol. The method has advantages in that the manufacturing cost is low and the waste PET as a raw material may be slightly contaminated, but has a disadvantage in that the molecular weight of a depolymerization material is high and it is difficult to be purified.

The hydrolysis method is a method which is frequently used for preparing terephthalic acid as a PET disintegration method by water, alkali, etc., and has a disadvantage, but has a disadvantage in that the manufacturing cost is large.

The methanolysis is most widely used as a depolymerization method by methanol, and has an advantage in that there is no relation with the contamination state of the waste PET, but has a disadvantage in that since it is necessary to operate at high temperatures and pressure, manufacturing costs are large.

Among the methods, since terephthalic acid may be used as a raw material of polyester fiber, a lot of studies on the hydrolysis method of separating terephthalic acid and ethylene glycol from the waste PET resin have been conducted.

The polyethylene terephthalate (PET) has advantages such as light weight, persistence, chemical stability, and

easy forming, and has been widely used in synthetic fibers, packaging, or the like. In 2015, the annual production of PET is about 330,000 tons, and in polyesters, a largest amount of PET has been produced, but the PET is difficult to become naturally disintegrated to cause environmental pollution, such as pollution of fine plastic, and plastic precipitation of marine ecosystem.

In order to replace the PET with eco-friendly plastic, biodegradable plastic such as polyethylene furandicarboxylic acid has been developed. However, these biodegradable plastics have many difficulties in replacing current PET in terms of physical properties or costs. For this reason, studies have been actively conducted to reduce waste plastics out of the nature by reinforcing the recycling of the PET.

Among many plastics, the PET and polyethylene are one of plastics which are physically recycled and may form recycled plastics. In the United States, recycling processes of PET have been performed for several decades, but the recycling rate is only 21%. This is because the recycled PET has less quality than newly produced PET and the cost of the recycling process is expensive. For example, the newly produced PET is 1.1 to 1.3 dollars per kilogram, but the cost of recycling PET is 1.3 to 1.5 dollars per kilogram.

Meanwhile, chemical or thermochemical plastic recycling of changing plastic into gas of fuel is not widely used due to energy cost and low price of products as compared with plastics. In summary, the recycling methods which have been currently used are not preferable solutions to reduce the waste PET. For this reason, recycling methods are required to upcycle the recycling method of the PET, that is, make a high value-added material using recycled articles.

2-pyrone-4,6-dicarboxylic acid (PDC) has a structure in which two carboxylic acid group are attached to a polar like-aromatic ring and acts as a difunctional monomer capable of polyaddition and polycondensation, and thus, high value-added utilization such as monomers useful for the production of various functional polyesters has been proposed. The 2-pyrone-4,6-dicarboxylic acid (PDC) is mass-produced enzymatically from lignin and is generally purified to activated carbon, but for high value-added utilization, many studies have been conducted by requiring a technology capable of purified the PDC to a high purity.

However, in other 2-pyrone-4,6-dicarboxylic acids commercially acceptable as well as 2-pyrone-4,6-dicarboxylic acids purified by existing methods, as NMR and TLC analysis results, it was estimated that a trace of impurities are contained. However, these impurities are hardly removed by conventional methods (e.g., recrystallization, cation precipitation method, solubility method, etc.) of purifying 2-pyrone-4,6-dicarboxylic acid, so that the high value-added utilization of 2-pyrone-4,6-dicarboxylic acid has been limited in terms of industrial use. In addition, the removal and purification thereof require a lot of costs and efforts, so that there is a need for a method of removing the impurities simply, efficiently, and economically.

## [Disclosure]

### [Technical Problem]

An object of the present invention is to provide high-purity PDC by producing terephthalic acid (TPA) from polyethylene terephthalate (PET) using a biomass catalyst, producing 2-pyrone-4,6-dicarboxylic acid (PDC) by reacting the TPA with a recombinant strain, and removing impurities of the PDC.

Another object of the present invention is to provide a method of easily producing solid terephthalic acid by producing a $SiO_2$ catalyst using rice hulls and reacting with polyethylene terephthalate in a microwave reactor using the produced $SiO_2$ catalyst.

Yet another object of the present invention is to provide a method of producing high-purity terephthalic acid by lowering the impurity content of terephthalic acid when hydrolyzing polyethylene terephthalate under neutral conditions.

Still another object of the present invention is to provide a recombinant strain for producing 2-pyrone-4,6-dicarboxylic acid (PDC) introduced with nucleotide sequences that encode TphB, TphAabc, LigAB, and LigC enzymes.

Still yet another object of the present invention is to provide a method of producing PDC including reacting a recombinant strain for producing the PDC with a substrate.

Still yet another object of the present invention is to provide high-purity 2-pyrone-4,6-dicarboxylic acid by removing impurities which are confirmed by NMR and/or TLC analysis, but hardly removed and purified, from crude 2-pyrone-4,6-dicarboxylic acid or its sodium salt which is industrially produced or commercially available.

The objects to be solved by the present disclosure are not limited to the aforementioned object(s), and other object(s), which are not mentioned above, will be apparent to those skilled in the art from the following description.

### [Technical Solution]

The present invention relates to a method of producing high-purity 2-pyrone-4,6-dicarboxylic acid (PDC), and a method of producing an intermediate for the same, the methods including the steps of: 1) producing TPA from PET; 2) producing PDC by reacting the TPA with a recombinant strain; and 3) purifying the produced PDC to a high purity.

According to the present invention, in the 1) producing of the TPA from PET, the $SiO_2$ catalyst may be extracted from a biomass.

The biomass is preferably rice hulls.

The $SiO_2$ catalyst has preferably a surface modified by a thiol functional group (-SH).

The specific surface area of the $SiO_2$ catalyst is preferably 151.92 to 281.02 $m^2/g$, and the size of the pore is preferably 0.57 to 0.66 nm.

According to another preferred embodiment of the present invention, a method of producing a biomass-derived $SiO_2$ catalyst may include a) grinding and then acid-treating rice hulls; and b) heat-treating the acid-treated rice hulls in step a).

In another embodiment, the acid-treating in step a) is preferably performed for 10 minutes to 3 hours at 40°C to 150°C using an acidic solution containing one selected from the group consisting of sulfuric acid, hydrochloric acid, nitric acid, phosphoric acid, and mixtures thereof.

After the acid-treating in step a), the method may further include filtering and washing the acid-treated rice hulls.

The heat-treating in step b) may be performed for 2 to 3 hours while lowering by at a rate of 3.5°C per minute at a temperature of 600°C to 700°C.

After step b), the method may further include modifying the surface of the $SiO_2$ catalyst, and the modifying of the surface of the $SiO_2$ catalyst may include preparing a mixture by adding the $SiO_2$ catalyst and mercaptopropyl trimethoxysilane in a toluene solvent; washing the mixture; and drying the washed mixture.

According to yet another preferred embodiment of the present invention, a disintegration method from PET to TPA using a biomass-derived $SiO_2$ catalyst is characterized by mixing polyethylene terephthalate, a biomass-derived $SiO_2$ catalyst, and water and irradiating microwaves to hydrolyze the polyethylene terephthalate to terephthalic acid.

In yet another embodiment, the method of hydrolyzing the polyethylene terephthalate to terephthalic acid may include a) preparing a mixture with precipitated terephthalic acid by reacting polyethylene terephthalate, a biomass-derived $SiO_2$ catalyst, and water in a microwave reactor; b) dissolving the terephthalic acid by mixing sodium hydroxide in the mixture; c) removing residues and the catalyst by filtering the mixture with the dissolved terephthalic acid; d) forming and obtaining a precipitate by adding an acidic solution to the mixture from which the residues and the catalyst are removed; and e) drying the obtained precipitate.

The acidic solution is preferably one selected from the group consisting of sulfuric acid, hydrochloric acid, nitric acid, phosphoric acid, and mixtures thereof.

An embodiment of 2) the producing of the PDC by reacting the TPA with the recombinant strain of the present invention provides a recombinant strain for producing 2-pyrone-4,6-dicarboxylic acid (PDC) introduced with nucleotide sequences which encode an enzyme selected from the group consisting of 1,2-dihydroxy-3,5-cyclohexadiene-1,4-dicarboxylate dehydrogenase (TphB) enzyme; terephthalate 1,2-dioxyganase (TphAabc) enzyme; protocatechuate 4,5-dioxyganase (LigAB) enzyme; 4-carboxy-2-hydroxymuconate-6-semialdehyde dehydrogenase (LigC) enzyme, and combinations thereof.

A nucleotide sequence *tphB* encoding the TphB enzyme is not particularly limited, but as an example, may consist of a nucleotide sequence of SEQ ID NO: 1; and a nucleotide sequence *tphAabc* encoding the TphAabc enzyme is not particularly limited, but as an example, may consist of a nucleotide sequence of SEQ ID NO: 2. A nucleotide sequence *ligAB* encoding the LigAB enzyme is not particularly limited, but as an example, may consist of a nucleotide sequence of SEQ ID NO: 3; and a nucleotide sequence *LigC* encoding the LigC enzyme is not particularly limited, but as an example, may consist of a nucleotide sequence of SEQ ID NO: 4.

As a detailed example of the embodiment, the recombinant strain for producing the PDC of the present invention may be a recombinant strain introduced with *tphB* of nucleotide SEQ ID NO: 1, *tphAabc* of nucleotide SEQ ID NO: 2, *ligAB* of nucleotide SEQ ID NO: 3, and *ligC* of nucleotide SEQ ID NO: 4; a mixed strain including a first recombinant strain introduced with *tphB* of nucleotide SEQ ID NO: 1 and *tphAabc* of nucleotide SEQ ID NO: 2 and a second recombinant strain introduced with *ligAB* of nucleotide SEQ ID NO: 3 and *ligC* of nucleotide SEQ ID NO: 4; and a recombinant strain introduced with *ligAB* of nucleotide SEQ ID NO: 3 and *ligC* of nucleotide SEQ ID NO: 4, etc.

The introduction of the nucleotide sequence may be performed through an expression vector, and the strain introduced with the nucleotide sequence is transformed so that the nucleotide sequence is expressed.

The expression vector refers to a gene construct including an essential regulating element operably linked to express a gene insert (the polynucleotide), as a vector capable of expressing a target protein or target RNA in a suitable host cell. The expression vector may be replicated regardless of host chromosome DNA once in the host cell, and inserted foreign DNA may be expressed. Since plasmid is the most commonly used form of the current vector, the plasmid and the vector are sometimes used interchangeably in the specification of the present invention.

The vector includes a plasmid vector, a cosmid vector, a bacteriophage vector, a viral vector, etc., but is not limited thereto. The suitable expression vector includes a signal sequence or a leader sequence for membrane targeting or secretion in addition to expression regulatory elements such as a promoter, an operator, an initiation codon, a termination codon, a polyadenylation signal, and an enhancer and may be variously manufactured according to a purpose.

The promoter of the vector may be constitutive or inductive. Further, the expression vector includes a selective marker for selecting a host cell containing a vector and a replicable expression vector includes a replication origin.

The transformation means that the DNA is introduced into a host to allow DNA to be replicated as an extrachromosomal factor or by chromosomal integration completion. The host cell that may be used in transformation according to the present invention may include all prokaryotic or eukaryotic cells, and hosts with high introduction efficiency of DNA and high expression efficiency of the introduced DNA may be used. For example, the host cells may use known prokaryotic or eukaryotic cells such as Escherichia, Pseudomonas, Bacillus, Streptomaces, fungi, and yeast, insect cells such as Spodoptera frugiperda (SF9), animal cells such as CHO, COS 1, COS 7, BSC 1, BSC 40, and BMT 10, etc., and are not limited thereto.

The transformation includes any method of introducing polynucleotides, and may be performed by selecting a suitable standard technique according to a host cell as known in the art. The method includes electroporation, protoplast fusion, calcium phosphate ($CaPO_4$) precipitation, calcium chloride ($CaCl_2$) precipitation, stirring using silicon carbide fibers, agrobacteria-mediated transformation, polyethylene glycol (PEG), dextran sulfate, lipofectamine, and particle bombardment, but is not limited thereto.

Another aspect of the present invention provides a method of producing PDC including reacting a recombinant strain for producing the PDC with a substrate.

The method of producing the PDC provided in the present invention is not particularly limited thereto. As a detailed example, the method of producing the PDC includes (1) obtaining a recombinant strain introduced with a nucleotide sequence of SEQ ID NO: 1 encoding a TphB enzymes, a nucleotide sequence of SEQ ID NO: 2 encoding a TphAabc enzyme, a nucleotide sequence of SEQ ID NO: 3 encoding a LigAB enzyme, and a nucleotide sequence of SEQ ID NO: 4 encoding a LigC enzyme; and (2) producing 2-pyrone-4,6-dicarboxylic acid (PDC) by reacting the obtained recombinant strain with terephthalic acid (TPA).

At this time, a catalytic reaction of converting terephthalic acid (TPA) into protocatechuic acid (PCA) may be performed through sequential reaction of the TphAabc enzyme and the TphB enzyme. The LigAB enzyme may perform a catalytic reaction of converting protocatechuic acid (PCA) into 4-carboxy-2-hydroxymuconate-6-semialdehyde (CHMS) and the LigC enzyme may perform a catalytic reaction of converting CHMS into PDC.

As another example, the method of producing the PDC provided by the present invention includes (1) obtaining a first recombinant strain introduced with a nucleotide sequence of SEQ ID NO: 1 encoding a TphB enzymes and a nucleotide sequence of SEQ ID NO: 2 encoding a TphAabc enzyme and a second recombinant strain introduced with a nucleotide sequence of SEQ ID NO: 3 encoding a LigAB enzyme and a nucleotide sequence of SEQ ID NO: 4 encoding a LigC enzyme, respectively; (2) obtaining a mixed strain by mixing the obtained first recombinant strain and second recombinant strain at a ratio of 15:25 (cell number); and (3) producing 2-pyrone-4,6-dicarboxylic acid (PDC) by reacting the obtained mixed strain with terephthalic acid (TPA).

As yet another example, the method of producing the PDC provided by the present invention includes (1) obtaining a recombinant strain introduced with a nucleotide sequence of SEQ ID NO: 3 encoding a LigAB enzyme and a nucleotide sequence of SEQ ID NO: 4 encoding a LigC enzyme; and (2) producing 2-pyrone-4,6-dicarboxylic acid (PDC) by reacting the obtained recombinant strain with protocatechuic acid (PCA).

Further, in order to achieve the object, the present invention provides a method of purifying high-purity 2-pyrone-4,6-dicarboxylic acid including the following steps in the case of the step 3) of purifying the produced PDC to a high purity:

(a) preparing an aqueous solution containing 2-pyrone-4,6-dicarboxylic acid by acidifying crude 2-pyrone-4,6-dicarboxylic acid sodium salt;
(b) extracting and layer-separating the aqueous solution resulted in step (a) with an organic solvent;
(c) separating and removing a white precipitate formed by adding acetone to the layer-separated water layer in step (b);
(d) extracting and layer-separating the solution from which the white precipitate is removed in step (c) with an organic solvent;
(e) binding and concentrating the layer-separated organic layer in step (b) and the layer-separated organic layer in step (d); and
(f) column-chromatographing the concentrate resulted in step (e) with a mixed solvent of ethyl acetate and hexane.

According to an embodiment of the present invention, the 2-pyrone-4,6-dicarboxylic acid sodium salt may include one or more other metal cations selected from the group consisting of monovalent metal cations such as lithium, potassium, rubidium, silver, and cesium; bivalent metal cations such as magnesium, calcium, iron (II), copper (II), zinc, barium, cobalt, nickel (II), manganese, and chromium (II); trivalent metal cations such as iron (III), aluminum, and gallium; and tetravalent metal cations such as arsenic(IV), lead (IV), titanium (IV), and germanium (IV).

According to an embodiment of the present invention, the acidifying in step (a) may be performed using at least one acid selected from the group consisting of hydrochloric acid, sulfuric acid, nitric acid and phosphoric acid, preferably,

hydrochloric acid.

According to an embodiment of the present invention, the organic solvents used in steps (b) and (d) are the same as or different from each other, may be at least one or two selected from the group consisting of ethyl acetate, methyl ethyl ketone, THF and mixtures thereof, preferably ethyl acetate.

According to an embodiment of the present invention, the column chromatography of step (f) may be eluted with a mixed solvent of ethyl acetate:hexane = 4:1 to 1:4, and preferably gradient-eluted while being changed from the mixed solvent of ethyl acetate:hexane = 4:1 to 1:4 to ethyl acetate (single solvent).

According to an embodiment of the present invention, the column chromatography of step (f) may be silica gel column chromatography.

**[Advantageous Effects]**

According to the present invention, it is possible to provide high-purity PDC by producing terephthalic acid (TPA) from polyethylene terephthalate (PET) using a biomass catalyst, producing 2-pyrone-4,6-dicarboxylic acid (PDC) by reacting the TPA with a recombinant strain, and removing impurities of the PDC.

Further, by using a biomass-derived $SiO_2$ catalyst, a method of producing the same, and a disintegration method of from PET to TPA using the same according to the present invention, high-purity terephthalic acid (TPA) can be produced at a faster rate than a conventional PET hydrolysis rate under neutral conditions, wherein the produced TPA can be usefully utilized as a raw material for polyester fibers and the like.

Further, in the case of producing 2-pyrone-4,6-dicarboxylic acid (PDC) using the recombinant strain provided in the present invention, a conversion amount in a predetermined amount of substrate is 100%, only the PDC can be selectively produced without by-products, the separation and purification for the by-products are not required to be economic, and this may be widely used for economic production of PDC.

Further, according to the present invention, it is possible to simply remove impurities, which are confirmed by NMR and/or TLC analysis, but hardly removed and purified, at low costs, from crude 2-pyrone-4,6-dicarboxylic acid or its sodium salt which is industrially manufactured or commercially available.

**[Description of Drawings]**

FIG. 1 illustrates a method of producing a biomass-derived $SiO_2$ catalyst.
FIG. 2 illustrates a method of hydrolyzing polyethylene terephthalate to terephthalic acid.
FIG. 3 illustrates SEM images of Examples 1 and 2, where (a) to (f) are SEM images of Examples 1 and 2.
FIG. 4 illustrates an analysis of an X-ray diffraction pattern of an $SiO_2$ catalyst produced according to Example 1.
FIG. 5 illustrates results of measuring XPS of the $SiO_2$ catalysts produced according to Examples 1 and 2, where (a) to (f) are results of measuring XPS of the $SiO_2$ catalysts produced according to Examples 1 and 2.
FIG. 6 illustrates XPS quantitative analysis of the $SiO_2$ catalysts produced according to Examples 1 and 2, where (a) and (b) are XPS quantitative analysis of the $SiO_2$ catalysts produced according to Examples 1 and 2.
FIG. 7 illustrates $N_2$ adsorption/desorption isotherms of the $SiO_2$ catalysts produced according to Examples 1 and 2, where (a) and (b) are $N_2$ adsorption/desorption isotherms of the $SiO_2$ catalysts produced according to Examples 1 and 2.
FIG. 8 illustrates a $NH_3$-TPD measuring result of the $SiO_2$ catalysts produced according to Examples 1 and 2.
FIG. 9 is graphs showing the yield of TPA according to a time and a temperature, where (a) and (b) are graphs showing the yield of TPA according to a time and a temperature.
FIG. 10 illustrates NMR analysis of terephthalic acid (TPA), where (a) and (b) are NMR analysis of terephthalic acid (TPA).
FIG. 11 is graphs showing a reaction rate constant value for a concentration and a time, where (a) and (b) are graphs showing a reaction rate constant value for a concentration and a time.
FIG. 12 illustrates a result of converting whole cells using protocatechuic acid as a substrate by using a recombinant E. coli strain of Experimental Group 1 provided in the present invention and confirming CHMS converted therefrom.
FIG. 13 illustrates a result of converting whole cells using protocatechuic acid as a substrate by using a recombinant E. coli strain of Experimental Group 2 provided in the present invention and confirming 2-pyrone-4,6-dicarboxylic acid (PDC) converted therefrom.
FIG. 14 illustrates a result of converting whole cells using terephthalic acid as a substrate by using a recombinant E. coli strain of Experimental Group 3 provided in the present invention and confirming 2-pyrone-4,6-dicarboxylic acid (PDC) converted therefrom.
FIG. 15 illustrates a result of converting whole cells using terephthalic acid as a substrate by using a recombinant

E. coli strain of Experimental Group 4-1 and a recombinant E. coli strain of Experimental Group 4-2 provided in the present invention and confirming 2-pyrone-4,6-dicarboxylic acid (PDC) converted therefrom.

FIG. 16 illustrates an NMR graph of crude 2-pyrone-4,6-dicarboxylic acid sodium salt used in step (a) of the present invention.

FIG. 17 illustrates an NMR graph and a TLC analysis image of crude 2-pyrone-4,6-dicarboxylic acid sodium salt used in step (b) of the present invention.

FIG. 18 illustrates an NMR graph and a TLC analysis image of a white precipitate resulted in step (c) of the present invention.

FIGS. 19, 20, and 21 show NMR graphs of PDCs obtained in Comparative Examples 1, 2, and 3 of the present invention, respectively.

FIG. 22 illustrates an NMR graph and a TLC analysis image of PDC obtained by column chromatography in step (f) of the present invention.

**[Best Modes for the Invention]**

The present invention relates to a method of producing high-purity 2-pyrone-4,6-dicarboxylic acid (PDC), and a method of producing an intermediate for the same, the methods including the steps of: 1) producing TPA from PET; 2) producing PDC by reacting the TPA with a recombinant strain; and 3) purifying the produced PDC to a high purity.

A method of producing a biomass-derived $SiO_2$ catalyst may include a) grinding and then acid-treating rice hulls; and b) heat-treating the acid-treated rice hulls in step a).

The method of hydrolyzing the polyethylene terephthalate to terephthalic acid may include a) preparing a mixture with precipitated terephthalic acid by reacting polyethylene terephthalate, a biomass-derived $SiO_2$ catalyst, and water in a microwave reactor; b) dissolving the terephthalic acid by mixing sodium hydroxide in the mixture; c) removing residues and the catalyst by filtering the mixture with the dissolved terephthalic acid; d) forming and obtaining a precipitate by adding an acidic solution to the mixture from which the residues and the catalyst are removed; and e) drying the obtained precipitate.

Further, there is provided a method of producing PDC including reacting a recombinant strain for producing PDC with a substrate.

The method of producing the PDC provided in the present invention includes (1) obtaining a recombinant strain introduced with a nucleotide sequence of SEQ ID NO: 1 encoding a TphB enzymes, a nucleotide sequence of SEQ ID NO: 2 encoding a TphAabc enzyme, a nucleotide sequence of SEQ ID NO: 3 encoding a LigAB enzyme, and a nucleotide sequence of SEQ ID NO: 4 encoding a LigC enzyme; and (2) producing 2-pyrone-4,6-dicarboxylic acid (PDC) by reacting the obtained recombinant strain with terephthalic acid (TPA).

Further, the present invention provides a method of purifying high-purity 2-pyrone-4,6-dicarboxylic acid including (a) preparing an aqueous solution containing 2-pyrone-4,6-dicarboxylic acid by acidifying crude 2-pyrone-4,6-dicarboxylic acid sodium salt; (b) extracting and layer-separating the aqueous solution resulted in step (a) with an organic solvent; (c) separating and removing a white precipitate formed by adding acetone to the layer-separated water layer in step (b); (d) extracting and layer-separating the solution from which the white precipitate is removed in step (c) with an organic solvent; (e) binding and concentrating the layer-separated organic layer in step (b) and the layer-separated organic layer in step (d); and (f) column-chromatographing the concentrate resulted in step (e) with a mixed solvent of ethyl acetate and hexane.

**[Modes for the Invention]**

Hereinafter, preferred embodiments of the present invention will be described in detail with reference to the accompanying drawings.

Advantages and features of the present disclosure, and methods for accomplishing the same will be more clearly understood from exemplary embodiments to be described below in detail with reference to the accompanying drawings.

However, the present disclosure is not limited to the following exemplary embodiments but may be implemented in various different forms. The exemplary embodiments are provided only to make description of the present disclosure complete and to fully provide the scope of the present disclosure to a person having ordinary skill in the art to which the present disclosure pertains with the category of the invention, and the present disclosure will be defined by the appended claims.

In the following description of the present invention, a detailed description of known arts related thereto will be omitted when it is determined to make the subject matter of the present invention rather unclear.

Hereinafter, a preferable embodiment of the present invention will be described in detail with reference to the accompanying drawings.

The present invention relates to a method of producing high-purity 2-pyrone-4,6-dicarboxylic acid (PDC), and a method of producing an intermediate for the same, the methods including the steps of: 1) producing TPA from PET; 2) producing PDC by reacting the TPA with a recombinant strain; and 3) purifying the produced PDC to a high purity.

## 1. Production of TPA from PET

A biomass described in the present invention described in the description of the present invention may be rice hulls.
$SiO_2$ nanoparticles prepared from the biomass may be a $SiO_2$ catalyst.
Further, the $SiO_2$ catalyst with the surface modified by a thiol functional group is expressed as a Thiol-$SiO_2$ catalyst.
An object of the present invention is to provide a method capable of easily disintegrating solid terephthalic acid by preparing a $SiO_2$ catalyst using rice hulls and reacting with polyethylene terephthalate in a microwave reactor using the produced $SiO_2$ catalyst.
Another object of the present invention is to provide a method of producing high-purity terephthalic acid by lowering the impurity content of terephthalic acid when hydrolyzing polyethylene terephthalate under neutral conditions.
The present invention provides a $SiO_2$ catalyst extracted from the biomass and a preparation method, and provides a method of hydrolyzing polyethylene terephthalate using the $SiO_2$ catalyst.
The biomass according to an embodiment of the present invention may be rice hulls.
The $SiO_2$ catalyst according to an embodiment of the present invention may be extracted from the biomass, and the surface of the $SiO_2$ catalyst may be modified by a thiol functional group (-SH).
The specific surface area of the $SiO_2$ catalyst may be 151.92 to 281.02 $m^2$/g, and the size of the pore may be 0.57 to 0.66 nm.
Hereinafter, a method of producing a biomass-derived $SiO_2$ catalyst according to an embodiment of the present invention will be described with reference to FIG. 1.
FIG. 1 illustrates a method of producing a biomass-derived $SiO_2$ catalyst.
According to an embodiment of the present invention, a method of producing a biomass-derived $SiO_2$ catalyst may include a) grinding and then acid-treating rice hulls (S10); and b) heat-treating the acid-treated rice hulls in step a) (S20).
Step a) (S10) is a step of grinding and then acid-treating rice hulls.
The reason for grinding the rice hulls to a certain size is to increase the efficiency of the acid-treating process after grinding, and increase the purity of the finally produced $SiO_2$.
The method of grinding the rice hulls is not limited, and specifically, milling may be performed.
The milling may be performed using a generally used milling machine, such as a knee-type milling machine, a bed-type milling machine, a universal milling machine, and an upright milling machine.
The particle size of the milled rice hulls may be 50 to 500 $\mu$m.
The acid-treating of step a) (S10) is to remove impurities contained in the rice hulls, and may be performed with 7 to 15% of an acidic solution, specifically, a 10% acidic solution.
The acidic solution may be one selected from the group consisting of sulfuric acid, hydrochloric acid, nitric acid, phosphoric acid, and mixtures thereof, and specifically hydrochloric acid.
The acid-treating of step a) (S10) may be performed for 10 minutes to 3 hours at 40°C to 150°C using an acidic solution including one selected from the group consisting of sulfuric acid, hydrochloric acid, nitric acid, phosphoric acid, and mixtures thereof. Specifically, the acid-treating of step a) (S10) may be performed for 20 minutes to 2 hours at 50°C to 100°C using hydrochloric acid, and more specifically, may be performed for 30 minutes at 60°C using hydrochloric acid.
The acid-treating of the grinded rice hulls may be performed at least one time, specifically 1 to 10 times, and may be performed differently depending on a type and a concentration of the acidic solution.
The acid-treating of step a) (S10) may be performed at a solid-liquid ratio (g:ml) of 1:5 to 15 of the grinded rice hulls and the acidic solution, specifically at a solid-liquid ratio of 1:10.
After the acid-treating in step a) (S10), the method may further include filtering and washing the acid-treated rice hulls.
The filtering is not limited so long as the method is any method capable of obtaining $SiO_2$ nanoparticles.
The washing may be performed 1 to 4 times with distilled water, toluene, acetone, etc.
The step b) (S20) is a step of heat-treating the acid-treated rice hulls in step (a) (S10).
The heat-treating of step b) (S20) may be performed for 2 to 3 hours while lowering the temperature of the acid-treated rice hulls by at a rate of 3.5°C per minute at a temperature of 600°C to 700°C.
When the heat-treating is performed under the above conditions, a $SiO_2$ catalyst with a high specific surface area (151.92 ~ 281.02 $m^2$/g) and a large pore (0.57 to 0.66 nm) may be obtained.
After step b) (S10), the method may further include modifying the surface of the $SiO_2$ catalyst, and the modifying

of the surface of the $SiO_2$ catalyst may include preparing a mixture by adding the $SiO_2$ catalyst and mercaptopropyl trimethoxysilane in a toluene solvent; washing the mixture; and drying the washed mixture.

The washing of the mixture may be performed 1 to 4 times with distilled water, toluene, acetone, etc.

The drying of the washed mixture may be vacuum-dried for 12 to 26 hours at 50°C to 70°C.

FIG. 2 illustrates a method of hydrolyzing polyethylene terephthalate to terephthalic acid.

According to another embodiment of the present invention, a disintegration method from PET to TPA using a biomass-derived $SiO_2$ catalyst is characterized by mixing polyethylene terephthalate, a biomass-derived $SiO_2$ catalyst, and water and irradiating microwaves to hydrolyze the polyethylene terephthalate to terephthalic acid.

Specifically, the method of hydrolyzing polyethylene terephthalate to terephthalic acid using the biomass-derived $SiO_2$ catalyst may include a) preparing a mixture with precipitated terephthalic acid by reacting polyethylene terephthalate, a biomass-derived $SiO_2$ catalyst, and water in a microwave reactor (S100); b) dissolving the terephthalic acid by mixing sodium hydroxide in the mixture (S200); c) removing residues and the catalyst by filtering the mixture with the dissolved terephthalic acid (S300); d) forming and obtaining a precipitate by adding an acidic solution to the mixture from which the residues and the catalyst are removed (S400); and e) drying the obtained precipitate (S500).

The following Reaction Formula 1 illustrates a process of hydrolyzing polyethylene terephthalate to terephthalic acid.

[Chemical Formula 1]

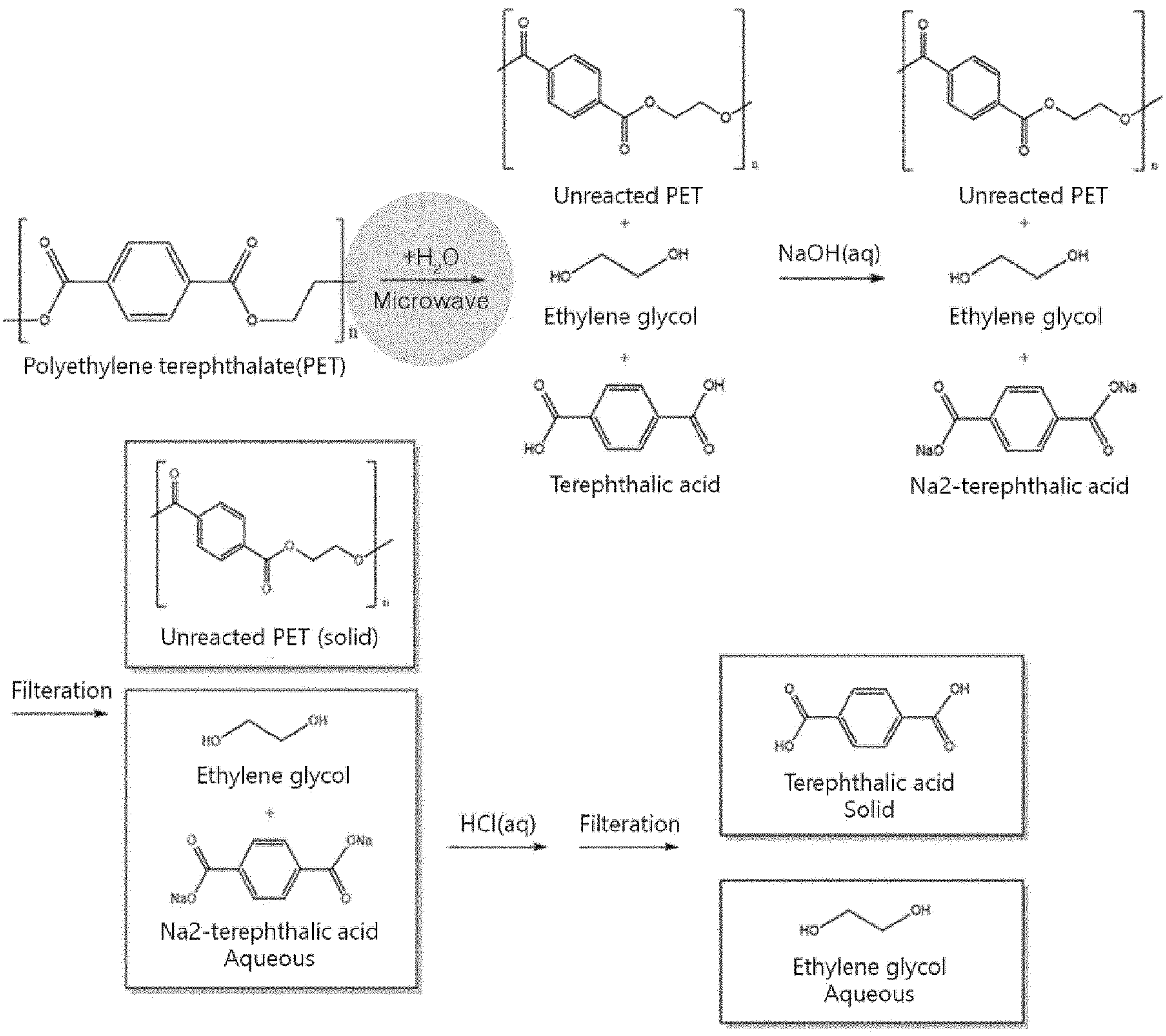

Polyethylene terephthalate(PET)
Unreacted PET + Ethylene glycol + Terephthalic acid
Unreacted PET + Ethylene glycol + Na2-terephthalic acid
Unreacted PET (solid)
Ethylene glycol + Na2-terephthalic acid Aqueous
Terephthalic acid Solid
Ethylene glycol Aqueous

Step a) (S100) is a step of hydrolyzing polyethylene terephthalate to terephthalic acid using the biomass-derived $SiO_2$ catalyst.

The hydrolyzing may be performed in a microwave reactor.

The microwave reactor (Anton Paar, Monowave 400, UK) may be equipped with temperature and pressure

sensors.

Since the description of the microwave reactor is beyond the scope of the present invention, the description thereof will be omitted.

The $SiO_2$ catalyst may be a biomass-derived $SiO_2$ catalyst and the biomass may be rice hulls.

Further, the biomass-derived $SiO_2$ catalyst may have a surface modified by a thiol functional group.

Since the $SiO_2$ catalyst has been described above, the description thereof will be omitted.

In order to hydrolyze the polyethylene terephthalate, since it is obvious that water is required, the description thereof will be omitted.

Referring to Reaction Formula 1, when the polyethylene terephthalate is hydrolyzed, a mixture in which terephthalic acid is precipitated is prepared.

The mixture may be terephthalic acid, ethylene glycol, polyethylene terephthalate, and a catalyst.

Step b) (S200) is a step of dissolving the terephthalic acid by mixing sodium hydroxide in the mixture.

In Step b) (S200), the terephthalic acid is dissolved to remove impurities in the next step.

Here, the impurities are unreacted polyethylene terephthalate and the catalyst.

Step c) (S300) is a step of removing residues and the catalyst by filtering the mixture with the dissolved terephthalic acid.

The residue is disintegrated polyethylene terephthalate.

Step c) (S300) is not limited so long as the method is any method capable of removing the disintegrated polyethylene terephthalate and the catalyst.

Step d) (S400) is a step of forming and obtaining a precipitate by adding an acidic solution to the mixture from which the residues and the catalyst are removed.

The acidic solution may be one selected from the group consisting of sulfuric acid, hydrochloric acid, nitric acid, phosphoric acid, and mixtures thereof, and preferably hydrochloric acid.

Step e) (S500) is a step of drying the obtained precipitate.

The method of drying the obtained precipitate is not limited.

Hereinafter, the present invention will be described in more detail by Examples and Experimental Examples. However, the following Examples and Experimental Examples are just illustrative of the present invention and the scope of the present invention is not limited thereto.

## Example 1: Production of biomass-derived $SiO_2$ catalyst

First, rice hulls were ground using a grinder and prepared so that the particle size of the rice hulls was 150 $\mu$m.

10 g of the ground rice hulls were immersed in 100 ml of a hydrochloric acid (10% HCl) aqueous solution for 15 hours.

Next, the hydrochloric acid aqueous solution was removed using a filter, and then the rice hulls were washed twice with distilled water.

Finally, the washed rice hulls were heat-treated (annealing) for 3 hours while falling by 3.5°C per minute at a temperature of 65°C to produce a biomass-derived $SiO_2$ catalyst.

## Example 2: Production of surface-modified $SiO_2$ catalyst

First, 0.5 g of the biomass-derived $SiO_2$ catalyst produced in Example 1 and 0.5 ml of mercaptopropyl trimethoxysilane were mixed in 200 ml of toluene and then refluxed for 24 hours and washed with toluene and acetone.

Finally, the mixture was dried for 24 hours in a vacuum oven of 60°C to produce a surface-modified $SiO_2$ (Thiol-$SiO_2$) catalyst.

## Experiment Example 1: Hydrolysis of non-catalvstic PET

First, 0.1 g of polyethylene terephthalate and 12 ml of deionized water were added in a microwave reactor (Anton Paar, Monowave 400, UK) to prepare a mixture in which terephthalic acid was precipitated.

After the reaction, 1 M of sodium hydroxide was added to the mixture in which terephthalic acid was precipitated and the precipitated terephthalic acid (TPA) was dissolved and filtered in a form of di-2-methyl terephthalic acid (NaTPA) to remove unreacted polyethylene terephthalate.

Next, the TPA was precipitated and filtered by adding an excess amount of hydrochloric acid to separate the TPA.

Finally, the dried TPA was obtained in an oven of 60°C.

**Experiment Example 2: Hydrolysis of PET using biomass-derived $SiO_2$ catalyst**

First, 0.1 g of the $SiO_2$ catalyst produced in Example 1, 0.1 g of polyethylene terephthalate and 12 ml of deionized water were added in a microwave reactor (Anton Paar, Monowave 400, UK) to prepare a mixture in which terephthalic acid was precipitated.
After the reaction, 1 M of sodium hydroxide was added to the mixture in which terephthalic acid was precipitated and the precipitated terephthalic acid (TPA) was dissolved and filtered in a form of di-2-methyl terephthalic acid (NaTPA) to remove unreacted polyethylene terephthalate and the $SiO_2$ catalyst.
Next, the TPA was precipitated and filtered by adding an excess amount of hydrochloric acid to separate the TPA.
Finally, the dried TPA was obtained in an oven of 60°C.

**Experiment Example 3: Hydrolysis of PET using surface-modified $SiO_2$ catalyst**

First, 0.1 g of the surface-modified $SiO_2$ catalyst produced in Example 2, 0.1 g of polyethylene terephthalate and 12 ml of deionized water were added in a microwave reactor (Anton Paar, Monowave 400, UK) to prepare a mixture in which terephthalic acid was precipitated.
After the reaction, 1 M of sodium hydroxide was added to the mixture in which terephthalic acid was precipitated and the precipitated terephthalic acid (TPA) was dissolved and filtered in a form of di-2-methyl terephthalic acid (NaTPA) to remove unreacted polyethylene terephthalate and the surface-modified $SiO_2$ catalyst.
Next, the TPA was precipitated and filtered by adding an excess amount of hydrochloric acid to separate the TPA.
Finally, the dried TPA was obtained in an oven of 60°C.

**Test Example 1: Analysis of characteristics of $SiO_2$ catalyst**

FIG. 3 illustrates SEM images of Examples 1 and 2, where (a) to (f) are SEM images of Examples 1 and 2.
Referring to the (a) to (c) in FIG. 3, it can be confirmed that the $SiO_2$ catalyst produced according to Example 1 consists of $SiO_2$ particles with small sizes of about 50 nm and has a bar shape with a fine interconnection structure.
Referring to the (d) to (f) in FIG. 3, it can be confirmed that the surface-modified $SiO_2$ catalyst produced according to Example 2 is surface-modified and then continuously maintains a fine shape.
As a result, it can be confirmed that this nanostructure is a unique form of the $SiO_2$ catalyst.

**Test Example 2: X-ray diffraction analysis of $SiO_2$ catalyst**

FIG. 4 illustrates an analysis of an X-ray diffraction pattern of the $SiO_2$ catalyst produced according to Example 1.
Referring to FIG. 4, a wide protrusion was shown in a portion wherein a $2\theta$ value was 20°, which coincides with an X-ray diffraction pattern of amorphous silica.

**Test Example 3: Analysis by X-ray photoelectron spectroscopy (XPS) of $SiO_2$ catalyst**

FIG. 5 illustrates results of measuring XPS of the $SiO_2$ catalysts produced according to Examples 1 and 2, where (a) to (f) are results of measuring XPS of the $SiO_2$ catalysts produced according to Examples 1 and 2.
Referring to the (a) to (c) in FIG. 5, the $SiO_2$ catalyst produced according to Example 1 had a single peak at 532.8 eV in an O Is spectrum, which corresponded to oxygen (O) in a Si-O-Si binding environment. Further, in a Si 2p spectrum, the $SiO_2$ catalyst produced according to Example 1 had a single peak at 103.5 eV, which corresponded to silicon (Si) in an Si-O binding environment. In addition, in a S 2p spectrum, the $SiO_2$ catalyst produced according to Example 1 did not have a peak corresponding to sulfur.
Referring to the (d) to (f) in FIG. 5, the $SiO_2$ catalyst produced according to Example 2 had a single peak at 532.9 eV in an O Is spectrum. This is a result of confirming that even after the surface of the catalyst was modified with a thiol functional group, the Si-O-Si binding is not changed.
However, it can be confirmed that in the Si 2p spectrum, the $SiO_2$ catalyst produced according to Example 2 is deconvoluted to one peak representing a Si-O bond (103.8 ev) and the other peak representing a Si-S bond (102.4 eV), which means that the surface of the catalyst was modified with a thiol functional group.
Further, it was confirmed that in the S 2p spectrum, the $SiO_2$ catalyst produced according to Example 2 had peaks corresponding to S 2p1/2 and 2p3/2 at 164.6 eV and 163.5 eV, respectively, which can be confirmed that the surface of the catalyst was modified with a thiol functional group.
FIG. 6 illustrate XPS quantitative analysis of the $SiO_2$ catalysts produced according to Examples 1 and 2, where (a) and (b) are XPS quantitative analysis of the $SiO_2$ catalysts produced according to Examples 1 and 2.
Referring to the (a) and (b) in FIG. 6, it was confirmed that in the $SiO_2$ catalyst produced according to Example

1, a silicon atomic ratio was 31.24% and an oxygen atomic ratio was 64.01%, which coincided with a conventional $SiO_2$ configuration. It was confirmed that in the $SiO_2$ catalyst produced according to Example 2, a silicon atomic ratio was 26.26%, an oxygen atomic ratio was 50.65%, and a sulfur atomic ratio was 2.78%.

The reason why the atomic ratios of the $SiO_2$ catalyst produced according to Example 2 are different from those of Example 1 is that the surface of the $SiO_2$ catalyst produced according to Example 2 was modified by a thiol functional group.

As the XPS analysis result, it can be seen that in the $SiO_2$ catalyst produced according to Example 2, the surface modification was performed well, and even if the surface of the $SiO_2$ catalyst produced according to Example 1 was modified, the initial structure was not affected.

**Test Example 4: $N_2$ adsorption/desorption analysis and $NH_3$-TPD analysis of $SiO_2$ catalyst**

FIG. 7 illustrates $N_2$ adsorption/desorption isotherms of the $SiO_2$ catalysts produced according to Examples 1 and 2, where (a) and (b) are $N_2$ adsorption/desorption isotherms of the $SiO_2$ catalysts produced according to Examples 1 and 2, and FIG. 8 illustrates a $NH_3$-TPD measuring result of the $SiO_2$ catalysts produced according to Examples 1 and 2.

Referring to the (a) and (b) in FIG. 7, all the $SiO_2$ catalysts produced according to Examples 1 and 2 had iv-type isotherms of forming Hysteresis loops in a relative pressure (P/PO) range of 0.4 to 1.0 due to mesoporous solids through multilayer adsorption and then capillary condensation.

The specific surface area of the $SiO_2$ catalyst was calculated by a Brunauer-Emmett-Teller (BET) theory (SBET).

The specific surface areas of the $SiO_2$ catalysts produced according to Examples 1 and 2 were calculated to 281.02 $m^2/g$ and 151.92 $m^2/g$..

In addition, the pore size of the $SiO_2$ catalyst was calculated by a Hgoorvath-Kawazoe (HK) method.

The pore sizes of the $SiO_2$ catalysts produced according to Examples 1 and 2 were calculated to 0.57 nm and 0.66 nm, respectively.

Through the two results, it can be seen that the surface area and the pore size of the $SiO_2$ catalyst are almost not changed during the thiol modifying process.

Referring to FIG. 8, the $SiO_2$ catalyst produced according to Example 1 had a $NH_3$ adsorption peak in a low-temperature region (LT) of 161.6°C, and the calculated acidic region concentration was 0.00838 mmol/g.

Further, the $SiO_2$ catalyst produced according to Example 2 had a $NH_3$ adsorption peak in a low-temperature region (LT) of 161.6°C, and the calculated acidic region concentration was 0.00838 mmol/g.

However, in the $SiO_2$ catalyst produced according to Example 2, the thiol functional group of the surface acted as the acidic region and thus another $NH_3$ adsorption peak was confirmed in a high-temperature region (HT) of 369.4°C, and the calculated acidic region concentration was 0.39319 mmol/g.

**Test Example 5: Calculation of yield of terephthalic acid (TPA) according to hydrolysis of polyethylene terephthalate (PET)**

The yield of terephthalic acid was calculated by hydrolyzing polyethylene terephthalate using the $SiO_2$ catalysts produced according to Examples 1 and 2.

FIG. 9 is graphs showing the yield of TPA according to a time and a temperature, where (a) and (b) are graphs showing the yield of TPA according to a time and a temperature.

The yield of TPA was calculated by the following Equation 1.

[Equation 1]

$$\text{TPA Yield}(\%) = \frac{\text{Experimentally produced TPA amount(g)}}{\text{Theoretically produced TPA amount(g)}} \times 100\ (\%)$$

$$= \frac{\text{Produced TPA amount(g)}}{\frac{\text{Degraded PET amount(g)}}{192.2(g/mol)} \times 163.13\ g/mol} \times 100(\%)$$

The (a) in FIG. 9 compares the hydrolysis yield of terephthalic acid according to a reaction time at a temperature of 230°C.

Referring to the (a) in FIG. 9, the hydrolysis of polyethylene terephthalate without using the catalyst was not

reacted for an initial 5 minutes and then reacted for 50 minutes, and as a result, the yield reached 91.74%.

However, the hydrolysis of polyethylene terephthalate using the $SiO_2$ catalyst produced according to Example 1 was performed for a reaction time of initial 5 minutes and then reacted for 40 minutes, and as a result, the yield reached 92.4%. Further, the hydrolysis reaction of polyethylene terephthalate using the $SiO_2$ catalyst produced according to Example 2 was performed for 30 minutes, and as a result, the yield reached 93.43%.

As a result, it was confirmed that as compared with the hydrolysis reaction of polyethylene terephthalate without using the catalyst, the hydrolysis time when using the $SiO_2$ catalyst produced according to Example 1 was shortened by 10 minutes and the hydrolysis time when using the $SiO_2$ catalyst produced according to Example 2 was further shortened by 10 minutes.

This is because the acidity of the $SiO_2$ catalyst according to Example 2 is higher than that of the $SiO_2$ catalyst according to Example 1, and the slowly increasing of the hydrolysis rate when the reaction starts is due to irregular chain separation of polyethylene terephthalate.

After the irregular chain separation of the polyethylene terephthalate, the hydrolysis rate was rapidly increased and the hydrolysis yield of terephthalic acid reached 97.06%.

Referring to the (b) in FIG. 9, it was confirmed that the hydrolysis of polyethylene terephthalate at 170°C or less did not start, and the yield of terephthalic acid was increased as the temperature was increased when the temperature increased. In addition, the yield reached 93.43% at a reaction temperature of 230°C.

As a result, it was confirmed that the hydrolysis of polyethylene terephthalate without using the catalyst was not performed at a temperature of 170°C or less, but in the case of using the $SiO_2$ catalysts produced according to Examples 1 and 2, even under the condition of 170°C or less, the hydrolysis of polyethylene terephthalate may be performed.

## Test Example 6: NMR analysis of terephthalic acid (TPA) according to hydrolysis of polyethylene terephthalate (PET)

FIG. 10 illustrates NMR analysis of terephthalic acid (TPA), where (a) and (b) illustrate are analysis of terephthalic acid (TPA).

Referring to the (a) in FIG. 10, terephthalic acid prepared using the $SiO_2$ catalyst produced according to Example 2 showed two peaks for a hydroxy proton and an aromatic single proton, and chemical shift values were 13.31 ppm and 8.03 ppm, respectively.

Referring to the (b) in FIG. 10, terephthalic acid produced using the $SiO_2$ catalyst produced according to Example 2 showed three peaks in a $^{13}C$ NMR spectrum, which were aromatic carbon, quaternary aromatic carbon and carbonyl carbon, respectively, and the chemical shift values were shown as 129.7 ppm, 134.8 ppm and 166.4 ppm, respectively.

The above result was confirmed to coincide with conventional terephthalic acid data.

## Test Example 7: Dynamic analysis of hydrolysis of polyethylene terephthalate (PET)

Terephthalic acid hydrolysis of polyethylene terephthalate followed Equation 2 below.

[Equation 2]

$$v = k[A]^2$$

Here, v represents a reaction rate, k represents a reaction rate constant, and [A] represents a concentration of polyethylene terephthalate.

The reaction rate constant (k) value of polyethylene terephthalate hydrolysis reaction was determined according to the following Equation 3 depending on the integration rate rule.

[Equation 3]

$$1/[A]_t - 1/[A]_0 = kt$$

Here, $[A]_t$ represents a polyethylene terephthalate concentration of a specific reaction time, $[A]_0$ represents an initial polyethylene terephthalate concentration, and t represents a specific reaction time.

FIG. 11 is graphs showing a reaction rate constant value for a concentration and a time, where (a) and (b) are graphs showing a reaction rate constant value for a concentration and a time.

Referring to the (a) in FIG. 11, when there is no catalyst (non-catalyst) and in the case of using catalysts according to Examples 1 and 2, the reaction rate constant (k) values were 0.56055, 0.76866 and 1.33406, respectively.

The hydrolysis using the $SiO_2$ catalyst produced according to Example 1 exhibited hydrolysis characteristics 1.37 times more improved than the non-catalystic hydrolysis, and the hydrolysis using the $SiO_2$ catalyst produced according to Example 2 exhibited hydrolysis characteristics 2.37 times more improved than the non-catalystic hydrolysis.

The reason why the hydrolysis characteristics using the $SiO_2$ catalyst produced according to Example 2 were improved was that the activation energy was lowered, and this was calculated according to the following Equation 4 (Arrhenius Equation) based on a reaction temperature control experiment.

[Equation 4]

$$k = Ae - Ea/RT$$

Here, k represents a rate constant, A represents a pre-exponential factor, Ea represents activation energy, R represents a gas constant, and T represents an absolute temperature.

Equation 4 above may be changed to the following Equation 5.

[Equation 5]

$$\ln(k) = \ln(A) - Ea/R(1/T)$$

Referring to the (b) in FIG. 11, the activation energy of the hydrolysis of polyethylene terephthalate by the $SiO_2$ catalyst produced according to Example 2 was calculated to 23.19 kJ/mol, and this value is reduced to half or more of the activation energy (56.8 kJ/mol) of the hydrolysis of polyethylene terephthalate.

**Test Example 8: Hydrolysis mechanism analysis of polyethylene terephthalate of $SiO_2$ catalyst produced according to Example 2**

The following Reaction Formula 2 illustrates a process of hydrolyzing polyethylene terephthalate to terephthalic acid.

[Chemical Formula 2]

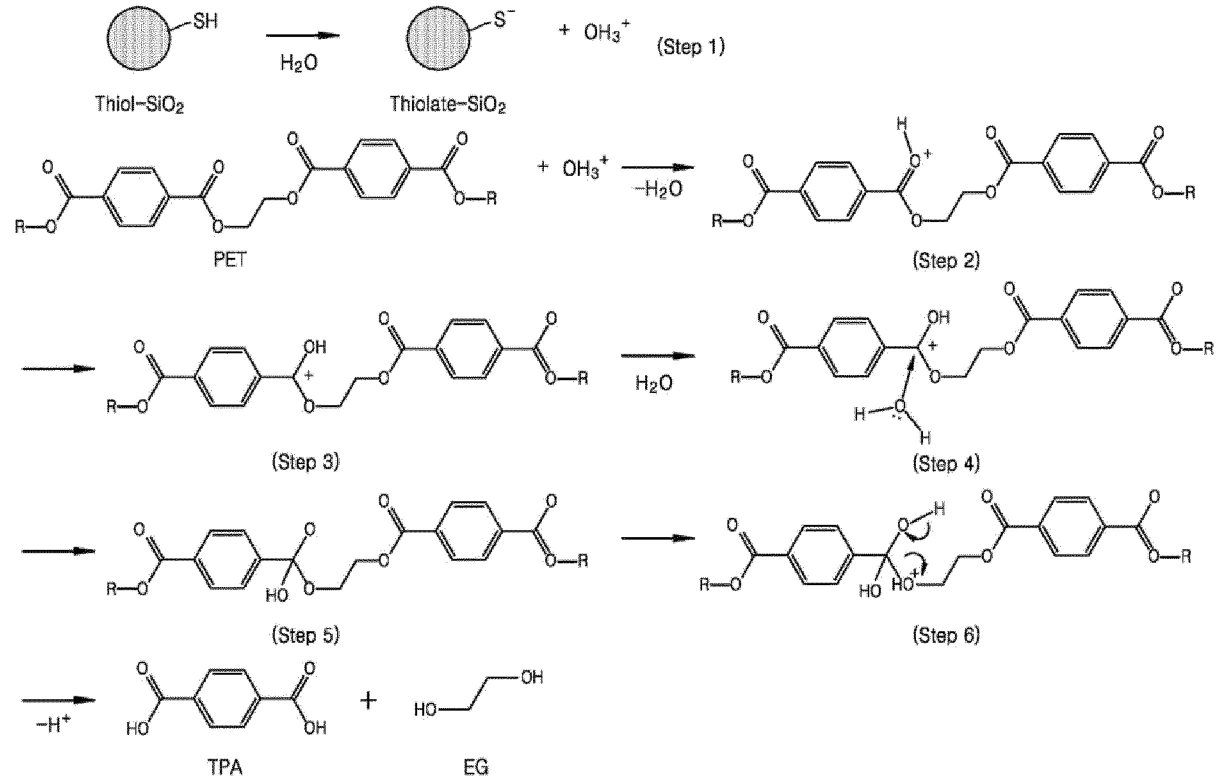


First, the thiol functional group of the surface of the $SiO_2$ catalyst according to Example 2 was easily deprotonated in an aqueous condition to be formed into thiolate and hydronium ($OH_3$+) ions.

Next, the generated hydronium ions provide protons in a nucleophilic center of polyethylene terephthalate.

In polyethylene terephthalate, oxygen atoms of an ester group are positively charged, but the positive charges are more delocalized in the central carbon atoms.

Next, water molecules attack positive-charged center carbon atoms, oxygen atoms of the ethyl group are provided with hydrogen atoms from other positive charged oxygen atoms, and the binding of carbons and oxygens are broken to generate terephthalic acid and ethylene glycol in a polyethylene terephthalate hydrolysis reaction.

If there is no $SiO_2$ catalyst produced according to Example 2, incomplete disintegration of ester bonds is induced from polyethylene terephthalate due to high activation energy to decrease the purity of terephthalic acid. The present invention solves the problems to produce high-purity terephthalic acid.

Therefore, by using a biomass-derived $SiO_2$ catalyst, a preparation method of the same, and a disintegration method from PET to TPA using the same according to the present invention, high-purity TPA can be produced at a faster time than the time of a conventional PET hydrolysis process under neutral conditions, wherein the TPA thus produced can be usefully utilized as a raw material for polyester fibers and the like.

## 2. Production of PDC by reacting TPA with recombinant strain

### Example 3: Construction of recombinant E. coli strain expression vector and Strain

In the present invention, in order to produce PDC from a recombinant strain, by using *Escherichia coli* (XL1-Blue) strain, there was constructed a recombinant E. coli strain expression vector introduced with a nucleotide sequence (SEQ ID NO: 1) encoding a 1,2-dihydroxy-3,5-cyclohexadiene-1,4-dicarboxylate dehydrogenase (TphB) enzyme, a nucleotide sequence (SEQ ID NO: 2) encoding a terephthalate 1,2-dioxyganase (TphAabc) enzyme, a nucleotide sequence (SEQ ID NO: 3) encoding protocatechuate 4,5-dioxyganase (LigAB) enzyme, and a nucleotide sequence (SEQ ID NO: 4) encoding 4-carboxy-2-hydroxymuconate-6-semialdehyde dehydrogenase (LigC) enzyme.

At this time, each nucleotide sequence encoding the TphB enzyme and the TphAabc enzyme was derived from *Comamonas sp.* E6 strain, and each nucleotide sequence encoding the LigAB enzyme and the LigC enzyme was derived from *Sphingomonas paucimobilis* strain.

[Table 1]

| Primer sequence | | Target gene |
|---|---|---|
| 1 | 5 '-ggatcc atgacaatagtgcaccgtagattg-3' | *tphB* |
| 2 | 5'-actagttt agaccggttgggctccg-3' | |
| 3 | Genetic synthesis request | *tphAabc* |
| 4 | | |
| 5 | Genetic synthesis request | *ligAB* |
| 6 | | |
| 7 | Genetic synthesis request | *ligC* |
| 8 | | |

As a specific method of constructing an expression vector, PKE112 plasmid was cleaved with restriction enzymes *Kpn*I and *Sbf*I and a *ligAB* gene cleaved with the restriction enzymes *Kpn*I and *Sbf*I was inserted to construct pKE112ligAB plasmid in Experimental Group 1. The constructed pKE112ligAB plasmid was cleaved with a restriction enzyme *Hind*III and a *ligC* gene cleaved with the restriction enzymes *Sbf*I and *Hind*III was inserted to construct pKE112ligABC in Experimental Group 2. In Experimental Group 3, pKE112 plasmid was cleaved with restriction enzymes *Ecor*I and *Kpn*I and a *tphB* gene cleaved with the restriction enzymes *Ecor*I and *Kpn*I was inserted.

Thereafter, in the same manner as Experimental Groups 1 and 2, a *ligABC* gene was inserted to construct pKE112tphBligABC plasmid. In the case of Experimental Group 3, pKM212 plasmid was cleaved with restriction enzymes *Kpn*I and *Hind*III and a *tphAabc* gene cleaved with the restriction enzymes *Kpn*I and *Hind*III was inserted to construct pKM212tphAabc. In the case of Experimental Group 4-1, like Experimental Group 3, a *tphB* gene was inserted to pKE112 plasmid to construct pKE112tphB. The pKM212tphAabc was constructed in the same manner as second plasmid of Experimental Group 3. In the case of Experimental Group 4-2, plasmid was the same plasmid as Experimental Group 2.

Thereafter, the expression vector was transformed into the E. coli strain to obtain the strains of Experimental Groups 1 to 4 of Table 2 below. In the case of Experimental Group 3, the two plasmids were transformed into the E. coli strain to obtain the strain. In the case of Experimental Group 4, the two plasmids of Experimental Group 4-1 were transformed into the E. coli strain to obtain the strain, and the plasmid of Experimental Group 4-2 was transformed into the E. coli strain to obtain the strain, and then whole-cell conversion was performed to a ratio of 15:25.

[Table 2]

| Condition | | Plasmid | |
|---|---|---|---|
| Experimental Group 1 | | pKE112ligAB | pKE112; $P_{tac}$ promoter, *Sphingomonas paucimobilis* strain *ligAB* gene, $Amp^R$ |
| Experimental Group 2 | | pKE112ligABC | pKE112; $P_{tac}$ promoter, *Sphingomonas paucimobilis* strain *ligABC* gene, $Amp^R$ |
| Experimental Group 3 | | pKE112tphBligABC | pKE112; $P_{tac}$ promoter,*Comamonas* sp. strain E6 *tphB* gene, *Sphingomonas paucimobilis* strain *ligABC* gene, $Amp^R$ |
| | | pKM212tphAabc | pKM212; $P_{tac}$ promoter,*Comamonas* sp. strain E6 *tphAabc* genes, $Km^R$ |
| Experimental Group 4 | 1 | pKE112tphB | pKE112; $P_{tac}$ promoter,*Comamonas* sp. strain E6 *tphB* genes, $Amp^R$ |
| | | pKM212tphAabc | pKM212; $P_{tac}$ promoter,*Comamonas* sp. strain E6 *tphAabc* genes, $Km^R$ |
| | 2 | pKE112ligABC | pKE112; $P_{tac}$ promoter, *Sphingomonas paucimobilis* strain *ligABC* gene, $Amp^R$ |

**Example 4: Preparation of CHMS from protocatechuic acid using recombinant E. coli strain**

The recombinant E. coli strain of Experimental Example 1 was inoculated in 14 ml of a round bottom tube including 2 ml of an LB medium (5 g/L yeast extract, 10 g/L of sodium chloride (NaCl), and 10 g/L of pancreatic enzyme hydrolysate (bacto-tryptone) of casein) and incubated for 8 hours under conditions of 37°C and 200 rpm.

Next, the culture solution incubated for 8 hours was added to a conical tube including 10 ml of an LB medium (5 g/L yeast extract, 10 g/L of sodium chloride (NaCl), and 10 g/L of pancreatic enzyme hydrolysate (bacto-tryptone) of casein), and then incubated overnight under conditions of 37°C and 200 rpm.

The culture solution incubated overnight was transferred to 2 L of a baffled Erlenmeyer flask including 500 ml of an LB medium (5 g/L yeast extract, 10 g/L of sodium chloride (NaCl), and 10 g/L of pancreatic enzyme hydrolysate (bacto-tryptone) of casein) again and then incubated under conditions of 37°C and 200 rpm until the cell growth was 0.4 at an absorbance (OD600). At this time, the incubating up to 0.4 at the absorbance (OD600) is for IPTG induction of the cells.

The IPTG induction is molecularly similar to allolactose to initiate transcription when a gene is under the control of lac operon. IPTG, an analog of allolactose, is not hydrolyzed by β-galactosidase unlike allolactos, and is maintained at the same concentration throughout an experimental environment. For this reason, the IPTG induction is used to induce protein expression.

For the IPTG induction, the culture solution was incubated until the cell growth became 0.4 at the absorbance (OD600) and then ice was added to stop the cell growth and added until the final concentration of IPTG became 0.1 mM and then incubated for about 24 hours under conditions of 16°C and 180 rpm until the cell growth stopped.

Next, the culture solution incubated for 24 hours was centrifuged for 20 minutes under conditions of 4°C and 8000 rpm for cell collection for whole-cell conversion. After centrifuging, the collected cells were washed with Tris-HCl at a concentration of 50 mM at pH 8.0 so that the cell concentration in a 20 ml volume was adjusted to 30 in the absorbance (OD600). The cells were centrifuged under the conditions of 4°C and 4400 rpm again and reacted under conditions of 30°C and 250 rpm in a 20 ml volume in a 250 ml baffled Erlenmeyer flask with 50 mM Tris-HCl of pH 8.0 containing 1 g/L of protocatechuic acid.

In order to measure CHMS converted from protocatechuic acid used as a substrate and recombinant E. coli by using a reaction solution of Experimental Group 1 reacted in the 250 ml baffled Erlenmeyer flask, HPLC analysis was performed under conditions of Table 3 and the results were illustrated in FIG. 12.

As illustrated in FIG. 12, when the whole-cell conversion was performed using protocatechuic acid as the substrate by using the recombinant E. coli strain, 1.20(±0.01) g/L of protocatechuic acid was fully consumed for 3 hours and the CHMS of a HPLC area value of 156.03(±9.63) was converted.

**Example 5: Preparation of 2-pyrone-4,6-dicarboxylic acid (PDC) from protocatechuic acid using recombinant E. coli strain**

The whole-cell conversion reaction of the recombinant E. coli strain of Experimental Group 2 was performed in the same manner as in Example 4, and in order to measure the CHMS converted from the protocatechuic acid used as the substrate and the recombinant E. coli and 2-pyrone-4,6-dicarboxylic acid (PDC), the HPLC analysis was performed under the conditions of the following Table 3 and the results were illustrated in FIG. 13.

As illustrated in FIG. 13, when the whole-cell conversion was performed using protocatechuic acid as the substrate by using the recombinant E. coli strain, 1.17(±0.01) g/L of protocatechuic acid was fully consumed for 6 hours and 2-pyrone-4,6-dicarboxylic acid (PDC) of the HPLC area value of 1313.80(±22.79) was converted.

**Example 6: Preparation of 2-pyrone-4,6-dicarboxylic acid (PDC) from terephthalic acid using recombinant E. coli strain**

The whole-cell conversion reaction of the recombinant E. coli strain of Experimental Group 3 was performed in the same manner as in Example 4, but a buffer (50 mM tris-HCl of PH 7.0 and 20 g/L glycerol) was used instead of a 50 mM Tris-HCl buffer of pH 8.0 and as the substrate, 0.5 g/L of terephthalic acid was used instead of 1 g/L of protocatechuic acid. In order to measure 2-pyrone-4,6-dicarboxylic acid (PDC) converted from terephthalic acid used as the substrate, protocatechuic acid and CHMS as intermediates, and the recombinant E. coli strain, the HPLC analysis was performed under the conditions of the following Table 3 and the results were illustrated in FIG. 14.

As illustrated in FIG. 14, when the whole-cell conversion was performed using terephthalic acid as the substrate by using the recombinant E. coli strain, 0.50(±0.01) g/L of terephthalic acid was fully consumed for 5 hours and 2-pyrone-4,6-dicarboxylic acid (PDC) of the HPLC area value of 1129.9(±17.4) was converted. The CHMS of the HPLC area value of 11.8(±2.3) was not converted to 2-pyrone-4,6-dicarboxylic acid (PDC) and then left in the whole-cell conversion solution.

**Example 7: Preparation of 2-pyrone-4,6-dicarboxylic acid (PDC) from terephthalic acid using two recombinant E. coli strains**

The whole-cell conversion reactions of the recombinant E. coli strain of Experimental Group 4-1 and the recombinant E. coli strain of Experimental Group 4-2 were performed in the same manner as in Example 4, but a ratio of the recombinant E. coli strain of Experimental Group 4-1 and the recombinant E. coli strain of Experimental Group 4-2 was 15:25. In order to measure 2-pyrone-4,6-dicarboxylic acid (PDC) converted from terephthalic acid used as the substrate, protocatechuic acid and CHMS as intermediates, and the recombinant E. coli strain, the HPLC analysis was performed under the conditions of the following Table 3 and the results were illustrated in FIG. 15.

[Table 3]

| | HPLC performance conditions | |
|---|---|---|
| | ODC and CHMS measurement condition | TPA and PCA measurement conditions |
| Column | Aminex HPX-87H column | Optimapak C18 column |
| Flow rate | 0.8 ml/min | 1.0 ml/min |
| Solvent | 5 mM of $H_2SO_4$ | A; 0.1 % trifluoroacetate in 100 % acetonitrile B; 0.1 % trifluoroacetate in waterA: 10 %B : 90% |
| Temperature | 50°C | 30°C |

As illustrated in FIG. 15, when the whole-cell conversion was performed using terephthalic acid as the substrate by using the recombinant E. coli strain of Experimental Group 4-1 and the recombinant E. coli strain of Experimental Group 4-2 at a ratio of 15:25, 0.52(±0.00) g/L of terephthalic acid was fully consumed for 6 hours and 2-pyrone-4,6-dicarboxylic acid (PDC) of the HPLC area value of 1585.73(±16.63) was converted. Unlike the result of FIG. 14, it can be seen that CHMS, which was an intermediate, has not been left in the whole-cell conversion solution, and thus, the CHMS, which was an intermediate, was fully converted.

**3. High-purity purification of produced PDC**

2-pyrone-4,6-dicarboxylic acid (hereinafter, abbreviated as PDC) was chemically stable and had a variety of functional groups, and thus was much expected as a multifunctional material. A pyrone ring of the PDC is a pseudo-aromatic ring, has a useful function such as receptor properties in donor-receptor type chromopores and biodegradable properties derived by PDC hydrolase, and two carboxylic acid functional groups attached to the pyrone ring are chemically modified when a large molecular system is made to act as a linker. Typically, the PDC has been studied to provide a linear or networked polymer by polycondensation with other difunctional or trifunctional monomers as difunctional monomers.

The PDC can not be synthesized using petrochemical technology, and currently, can be extracted only from plant ingredients and, for example, can be produced in a large scale by a microbial fermentation process.

When the PDC was produced by the microbial fermentation process, there has been proposed a method in which NaCl was added to a fermentation mixture containing the PDC to precipitate PDC sodium salt, and the PDC sodium salt was collected by centrifuge and acidified again, and then extracted with an organic solvent to purify pure PDC.

The PDC as a compound is very soluble in polar solvents such as water, acetone, methanol, THF and acetonitrile, but is hardly dissolved in non-polar solvents such as benzene, hexane and heptane. Most PDC metal salts such as PDC sodium salt have very low solubility in water or polar solvents, and then a PDC purification method has been developed using the same.

In the present invention, a method of producing or obtaining such crude PDC or crude PDC sodium salt is not particularly limited. Generally, the crude PDC or crude PDC sodium salt may be commercially available or prepared by the microbial fermentation process described in the prior art.

However, in the crude PDC or crude PDC sodium salt which is commercially available or prepared by the microbial fermentation process described in the prior art, it is analyzed that impurities are traced in a spectroscopic test of NMR, but in thin film chromatography (TLC) analysis, separate spots clearly distinguished from spots of PDC are observed. These spots are determined as impurities, and the amount thereof is also not small.

An object of the present invention is to provide an economic PDC purification method capable of removing these impurities simply and in low cost in the crude PDC or crude PDC sodium salt which is commercially available or

prepared by the microbial fermentation process described in the prior art.

The present invention provides a method of purifying 2-pyrone-4,6-dicarboxylic acid (PDC) including the following steps:

(a) preparing a crude PDC-containing aqueous solution by acidifying crude PDC sodium salt;
(b) extracting the crude PDC-containing aqueous solution resulted in step (a) with an organic solvent to be layer-separated into a water layer and an organic layer;
(c) separating and removing a white precipitate formed by adding acetone to the water layer resulted in step (b);
(d) extracting the solution from which the white precipitate is removed in step (c) with an organic solvent to be layer-separated into a water layer and an organic layer;
(e) concentrating the organic layer resulted in step (b) and the organic layer resulted in step (d); and
(f) column-chromatographing the concentrate resulted in step (e) with a mixed solvent of ethyl acetate and hexane.

In the present invention, Step (a) is a step of preparing an aqueous solution containing PDC by acidifying crude PDC sodium salt using at least one acid selected from the group consisting of, for example, hydrochloric acid, nitric acid, sulfuric acid, and phosphoric acid, as a step of preparing the crude PDC-containing aqueous solution.

In an embodiment of the present invention, the acidifying may be performed by adding the PDC sodium salt to water and intensively stirring to make a water dispersion or water mixture, and adding strong hydrochloric acid thereto or directly adding strong hydrochloric acid to the PDC sodium salt.

In an embodiment of the present invention, the acidifying may be performed by adding the PDC sodium salt to water and intensively stirring to make a water dispersion or water mixture, and adding strong hydrochloric acid thereto or directly adding strong hydrochloric acid to the PDC sodium salt.

In the accompanying drawings, FIGS. 16 and 17 show NMR graphs of crude PDC sodium salt and crude PDC, respectively, wherein peaks of two hydrogen atoms (around $\delta$ 7.0 to 7.4) of the pyrone ring are not shown in FIG. 16, but clearly shown in FIG. 17. In FIG. 17, peaks that suggest the presence of impurities are not clear, but peaks shown in $\delta$ 8 to 9 and $\delta$ 3 to 4 may be estimated as impurities.

On NMR graphs of FIGS. 16 and 17, the presence of impurities is not clear, but in a TLC image displayed on an upper end of FIG. 17, spots are clearly shown at other locations as well as PDC spots (marked as P) in a dotted line, and it is estimated that a large amount of impurities may be present from these spots.

In an embodiment of the present invention, the crude PDC sodium salt may be replaced with other metal cation salts or include some of the salts. Example of other metal cations may include other monovalent, bivalent, trivalenent, or tetravalent metal cations, specifically, monovalent metal cations such as lithium, potassium, rubidium, silver, and cesium; bivalent metal cations such as magnesium, calcium, iron (II), copper (II), zinc, barium, cobalt, nickel (II), manganese, and chromium (II); trivalent metal cations such as iron (III), aluminum, and gallium; and tetravalent metal cations such as arsenic(IV), lead (IV), titanium (IV), and germanium (IV).

The acidification of the crude PDC sodium salt may be performed using an inorganic acid such as hydrochloric acid, sulfuric acid, nitric acid, and phosphoric acid, and organic acids such as acetic acid, preferably hydrochloric acid, and more preferably strong hydrochloric acid. The solution containing the PDC acidified as such may have pH 6 or less, preferably pH 5 or less, and more preferably pH 3 to 5.

In the present invention, Step (b) is a step of extracting the crude PDC-containing aqueous solution resulted in step (a) with an organic solvent to be layer-separated into a water layer and an organic layer, and the extraction may be performed by adding an organic solvent to the crude PDC-containing aqueous solution and stirring, baffling, or shaking the mixture.

The organic solvent used in extraction is not particularly limited, but ethyl acetate, methyl ethyl ketone, THF, and the like may be mentioned, and preferably, ethyl acetate, THF, or a mixture thereof may be mentioned.

The amount of the organic solvent is not particularly limited, but generally, may be selected from 50 to 150 vol%, preferably 70 to 130 vol%, more preferably 90 to 110 vol% of the water layer.

Through NMR and TLC analysis, it was confirmed that trace impurities are contained in the PDC contained in the layer-separated organic layer (see FIG. 17).

In the present invention, step (c) is a step of separating and removing the white precipitate formed by adding acetone to the water layer layer-separated in step (b), and the water layer or the solution from the white precipitate is separated is obtained. As the NMR and TLC analysis results, the separated white precipitate did not contain the PDC and was confirmed as impurities.

The separation of the white precipitate is not particularly limited, and may use conventional solid solution separation methods, and for example, may be performed by filtration, centrifugation, gradient-separation, and the like.

In this regard, since PDC has high solubility in both water and acetone, but the white precipitate as impurities has high solubility in water, but has low solubility in acetone, when acetone is added to the water layer, the PDC is not precipitated and the impurities that are insoluble to acetone are precipitated.

According to one method of the present invention, other water-miscible organic solvents, such as methanol, ethanol, propanol, acetonitrile, dimethylformamide (DMF), dimethylsulfoxide (DMSO) and N-methylpyrrolidone (NMP) may be used instead of acetone or some thereof may be replaced.

The used amount of acetone or other water-miscible solvents is not particularly limited, but may be selected from 30 to 200 vol%, preferably 50 to 150 vol%, more preferably 60 to 120 vol%, specifically, 90 to 110 vol% of the water layer. When the used amount of acetone or other water-miscible solvents is in the range or more, the formation of the white precipitate may not be performed well, and when the used amount thereof is in the range or more, a process of removing some or all of water-miscible solvents used for a subsequent extraction process may be further required.

FIG. 18 is a diagram illustrating an NMR graph and a TLC analysis image (upper right end) of a white precipitate separated into filtration. When an NMR graph of the white precipitate shown in FIG. 18 is compared with the NMR graph (FIG. 16) of the PDC sodium salt and the NMR graph (FIG. 17) of the PDC, it is confirmed that the white precipitate is different from the PDC. Further, when the TLC analysis image of the white precipitate is compared with the TLC analysis image of the PDC (the upper left end of FIG. 17), it is confirmed that since the spot of the white precipitate is different from the position of the PDC spot and shown at the same position as one of spots of the PDC impurity, the white precipitate is the impurity other than the PDC.

In the present invention, Step (d) is a step of extracting the solution from which the white precipitate is separated in step (c) with an organic solvent to be layer-separated into a water layer and an organic layer, and the extraction may be performed by adding the organic solvent to the solution and stirring, baffling, or shaking the mixture.

The organic solvent used in extraction is not particularly limited, but ethyl acetate, methyl ethyl ketone, THF, and the like may be exemplified, and preferably, ethyl acetate, THF, or a mixture thereof may be mentioned.

The amount of the organic solvent is not particularly limited, but generally, may be selected from 50 to 150 vol%, preferably 70 to 130 vol%, more preferably 90 to 110 vol% of the water layer, and the solution may be extracted one or more times, preferably 2 to 3 times.

Step (e) of the present invention is a step of obtaining a concentrate containing 2-pyrone-4,6-dicarboxylic acid by concentrating the organic layer resulted in step (b) and the organic layers resulted in step (d).

According to a preferred embodiment of the present invention, the organic layer is dried in advance with magnesium sulfate, sodium sulfate, and the like, and then concentrated under reduced pressure or under normal pressure.

It is confirmed that in the PDC contained in the concentrate, as the NMR and TLC analysis results, a trace amount of impurities are still present.

Step (f) of the present invention is a step of column-chromatographing the concentrate resulted in step (e) with a mixed solvent of ethyl acetate and hexane, preferably, a mixed solvent of ethyl acetate:hexane = 4:1 to 1:4, and a step of obtaining 2-pyrone-4,6-dicarboxylic acid to high purity.

According to one embodiment of the present invention, silica gel column chromatography is used.

FIGS. 19, 20, and 21 illustrate NMR graphs and/or TLC analysis images of PDC obtained by separating the solid using a mixed solvent of ethyl acetate:methanol = 1:1, a mixed solvent of ethyl acetate:methanol = 2:1, and a single solvent of 100% ethyl acetate with column chromatography, respectively. Each NMR image suggests that impurities are trace, but not ignored in addition to PDC.

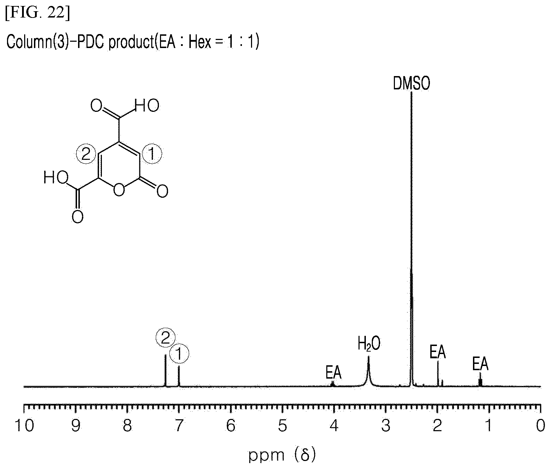

FIG. 22 shows an NMR graph of a 2-pyrone-4,6-dicarboxylic acid product obtained by separating the solid using a mixed solvent of ethyl acetate:methanol = 1:1 with column chromatography, wherein there is almost no peak corresponding to the impurities.

According to a preferred embodiment of the present invention, the column chromatography may be performed by isocratic elution or gradient elution. As an example of the gradient elution, it may be mentioned an elution method using finally using a single solvent of 100% ethyl acetate by starting from a mixed solvent of ethyl acetate and hexane selected from a ratio of 4:1 to 1:4 and then gradually increasing a ratio of ethyl acetate. In the present invention, gradient elution finally using a single solvent of 100% hexane by gradually decreasing a ratio of ethyl acetate is not excluded.

According to an advantage of the present invention, it is possible to provide high-purity 2-pyrone-4,6-dicarboxylic acid (PDC) by removing most impurities which are hardly confirmed and/or removed by a conventional purification method such as recrystallization, solvent precipitation, salting-out, and the like.

According to another advantage of the present invention, it is possible to provide high-purity 2-pyrone-4,6-dicarboxylic acid (PDC) capable of producing high-functional polymer in an industrial scale by an economic and simple method.

According to yet another advantage of the present invention, it is possible to provide a method of confirming and removing impurities which are contained in 2-pyrone-4,6-dicarboxylic acid (PDC) and hardly identified by a spectroscopic method such as NMR and/or a chromatographic method such as TLC.

## Preparation Example 1

A microbial culture solution (1.5 L) containing PDC was centrifuged (4000 rpm, 1 hour, 4°C) to remove cells.

15 g of NaCl was added to a supernatant and left for 12 hours at 4°C to precipitate PDC sodium salt, and the precipitated PDC sodium salt was collected by filtration to prepare crude PDC sodium salt.

The crude PDC sodium salt (22 g) was dispersed in water, acidified by adding strong hydrochloric acid, adjusted to pH 3 to 4, and concentrated under reduced pressure to prepare crude PDC.

FIG. 16 shows an NMR graph of crude PDC sodium salt and FIG. 17 shows an NMR graph and a TLC analysis image (deployment solvent chloroform: ethyl acetate:formamide = 10:8:1) of PDC obtained by acidifying crude PDC sodium salt with strong hydrochloric acid.

It is observed that two hydrogen atoms of the pyrone ring of PDC are not shown in the NMR graph in a sodium salt state (COONa state) (FIG. 16), but shown around $\delta$ 7.0 to 7.3 in an acidified state (COOH state) (FIG. 17). However, in the TLC analysis image of FIG. 17, it is shown that separate other spots as well as PDC spots (spots marked by P in a red dotted rectangle) are clearly shown and a large amount of impurities are included in addition to the PDC.

## Example 8

22 g of the crude PDC sodium salt obtained in Preparation Example 1 was dissolved in 100 ml of distilled water, slowly added with 1 M hydrochloric acid, adjusted to pH 3 to 4, and then extracted once with 100 ml of ethyl acetate (first extraction).

A water layer and an organic layer were separated and 30 ml of acetone was added to the water layer to precipitate a white solid, the formed white solid was removed by filtration, the remaining acetone was removed under reduced pressure, and then a filtrate was extracted twice with 100 ml of ethyl acetate again (second extraction).

The organic layers (ethyl acetate layers) obtained in the first extraction and the second extraction were bound and dried with anhydrous sulfuric acid magnesium, and the anhydrous sulfuric acid magnesium was removed by filtration to concentrate the resulted filtrate under reduced pressure.

The resulted concentrate was eluded with a mixed solvent of ethyl acetate:hexane = 1:1 in silica gel column chromatography and concentrated under reduced pressure to obtain high-purity PDC, and the yield for the reactant was 80% or more. In the fractions eluted in column chromatography, the PDC-containing was confirmed by TLC (ethyl acetate:hexane = 1:1).

## Example 9

Except that the concentrate obtained by concentrating the organic layer was gradient-eluted while changing slowly a ratio to an ethyl acetate signal solvent from a mixed solvent of ethyl acetate:hexane = 1:1 in silica gel column chromatography, Example 9 was performed in the same manner as Example 8.

In the resulted 2-pyrone-4,6-dicarboxylic acid, it was confirmed that most of impurities were removed through the NMR graph (FIG. 22).

## Comparative Example 1

Except that the concentrate obtained by concentrating the organic layer was eluted with a mixed solvent of ethyl acetate:hexane = 1:1 in silica gel column chromatography, Comparative Example 1 was performed in the same manner as Example 8.

In the resulted 2-pyrone-4,6-dicarboxylic acid, it was confirmed that a trace amount of impurities was still present through the NMR graph (FIG. 19).

## Comparative Example 2

Except that the concentrate obtained by concentrating the organic layer was eluted with a mixed solvent of ethyl acetate:hexane = 2:1 in silica gel column chromatography, Comparative Example 2 was performed in the same manner as Example 8.

In the resulted 2-pyrone-4,6-dicarboxylic acid, it was confirmed that a trace amount of impurities was still present through the NMR graph (FIG. 20).

## Comparative Example 3

Except that the concentrate obtained by concentrating the organic layer was eluted with ethyl acetate (single solvent) in silica gel column chromatography, Comparative Example 3 was performed in the same manner as Example 8.

In the resulted 2-pyrone-4,6-dicarboxylic acid, it was confirmed that a trace amount of impurities was still present through the NMR graph (FIG. 21).

As described above, specific embodiments for the method of producing high-purity 2-pyrone-4,6-dicarboxylic acid according to an embodiment of the present invention and the method of producing the intermediate for same have been described, but it will be apparent that various modification can be made without departing from the scope of the present invention.

Therefore, the scope of the present invention should not be limited to the embodiments and should be defined by the appended claims and equivalents to the appended claims.

In other words, the embodiments described above are illustrative in all aspects and should be understood as not being restrictive, and the scope of the present invention is represented by appended claims to be described below rather than the detailed description, and it is to be interpreted that the meaning and scope of the appended claims and all changed or modified forms derived from the equivalents thereof are included within the scope of the present invention.

## Claims

1. A method of producing high-purity 2-pyrone-4,6-dicarboxylic acid and an intermediate for the same comprising the steps of:

   1) producing terephthalic acid (TPA) from polyethylene terephthalate (PET);
   2) producing 2-pyrone-4,6-dicarboxylic acid (PDC) by reacting the TPA with a recombinant strain; and
   3) purifying the produced PDC to a high purity.

2. A $SiO_2$ catalyst, which is extracted from a biomass.

3. The $SiO_2$ catalyst of claim 2, wherein the biomass is rice hulls.

4. The $SiO_2$ catalyst of claim 2, wherein a surface is modified by a thiol functional group (-SH).

5. The $SiO_2$ catalyst of claim 2, wherein the specific surface area of the $SiO_2$ catalyst is 151.92 to 281.02 $m^2/g$, and the size of the pore is 0.57 to 0.66 nm.

6. A method of producing a biomass-derived $SiO_2$ catalyst comprising the steps of:

가 ) grinding and then acid-treating rice hulls; and

나 ) heat-treating the acid-treated rice hulls in step 가).

**7.** The method of producing the biomass-derived $SiO_2$ catalyst of claim 6, wherein the acid-treating in step 가) is performed for 10 minutes to 3 hours at 40°C to 150°C using an acidic solution containing one selected from the group consisting of sulfuric acid, hydrochloric acid, nitric acid, phosphoric acid, and mixtures thereof.

**8.** The method of producing the biomass-derived $SiO_2$ catalyst of claim 6, further comprising:

after the acid-treating in step 가), filtering and washing the acid-treated rice hulls.

**9.** The method of producing the biomass-derived $SiO_2$ catalyst of claim 6, wherein the heat-treating in step (나) is performed for 2 to 3 hours while lowering by at a rate of 3.5°C per minute at a temperature of 600°C to 700°C.

**10.** The method of producing the biomass-derived $SiO_2$ catalyst of claim 6, further comprising:

modifying the surface of the $SiO_2$ catalyst after step (나),
wherein the modifying of the surface of the $SiO_2$ catalyst comprises the steps of:

preparing a mixture by adding the $SiO_2$ catalyst and mercaptopropyl trimethoxysilane in a toluene solvent;
washing the mixture; and
drying the washed mixture.

**11.** A disintegration method from PET to TPA using a biomass-derived $SiO_2$ catalyst comprising:
mixing polyethylene terephthalate, a biomass-derived $SiO_2$ catalyst, and water and irradiating microwaves to hydrolyze the polyethylene terephthalate to terephthalic acid.

**12.** The disintegration method from PET to TPA using the biomass-derived $SiO_2$ catalyst of claim 11, wherein the method of hydrolyzing the polyethylene terephthalate to terephthalic acid comprises the steps of:

(a) preparing a mixture with precipitated terephthalic acid by reacting polyethylene terephthalate, a biomass-derived $SiO_2$ catalyst, and water in a microwave reactor;
(b) dissolving the terephthalic acid by mixing sodium hydroxide in the mixture;
(c) removing residues and the catalyst by filtering the mixture with the dissolved terephthalic acid;
(d) forming and obtaining a precipitate by adding an acidic solution to the mixture from which the residues and the catalyst are removed; and
(e) drying the obtained precipitate.

**13.** The disintegration method from PET to TPA using the biomass-derived $SiO_2$ catalyst of claim 12, wherein the acidic solution is one selected from the group consisting of sulfuric acid, hydrochloric acid, nitric acid, phosphoric acid, and mixtures thereof.

**14.** A recombinant strain for producing 2-pyrone-4,6-dicarboxylic acid (PDC) introduced with a nucleotide sequence which encodes an enzyme selected from the group consisting of 1,2-dihydroxy-3,5-cyclohexadiene-1,4-dicarboxylate dehydrogenase (TphB) enzyme; terephthalate 1,2-dioxyganase (TphAabc) enzyme; protocatechuate 4,5-dioxyganase (LigAB) enzyme; 4-carboxy-2-hydroxymuconate-6-semialdehyde dehydrogenase (LigC) enzyme, and combinations thereof.

**15.** The recombinant strain for producing the PDC of claim 14, wherein the nucleotide sequence encoding the TphB enzyme is represented by SEQ ID NO: 1.

**16.** The recombinant strain for producing the PDC of claim 14, wherein the nucleotide sequence encoding the TphAabc enzyme is represented by SEQ ID NO: 2.

**17.** The recombinant strain for producing the PDC of claim 14, wherein the nucleotide sequence encoding the LigAB

enzyme is represented by SEQ ID NO: 3.

**18.** The recombinant strain for producing the PDC of claim 14, wherein the nucleotide sequence encoding the LigC enzyme is represented by SEQ ID NO: 4.

**19.** A method of producing PDC comprising the steps of:

(a) obtaining a recombinant strain introduced with a nucleotide sequence of SEQ ID NO: 1 encoding a TphB enzymes, a nucleotide sequence of SEQ ID NO: 2 encoding a TphAabc enzyme, a nucleotide sequence of SEQ ID NO: 3 encoding a LigAB enzyme, and a nucleotide sequence of SEQ ID NO: 4 encoding a LigC enzyme; and
(b) producing 2-pyrone-4,6-dicarboxylic acid (PDC) by reacting the obtained recombinant strain with terephthalic acid (TPA).

**20.** The method of producing the PDC of claim 19, wherein the TphAabc enzyme and the TphB enzyme perform a catalytic reaction of converting terephthalic acid (TPA) into protocatechuic acid (PCA).

**21.** The method of producing the PDC of claim 19, wherein the LigAB enzyme performs a catalytic reaction of converting protocatechuic acid (PCA) into CHMS.

**22.** The method of producing the PDC of claim 19, wherein the LigC enzyme performs a catalytic reaction of converting CHMS into PDC.

**23.** A method of producing PDC comprising the steps of:

(a) obtaining a first recombinant strain introduced with a nucleotide sequence of SEQ ID NO: 1 encoding a TphB enzymes and a nucleotide sequence of SEQ ID NO: 2 encoding a TphAabc enzyme and a second recombinant strain introduced with a nucleotide sequence of SEQ ID NO: 3 encoding a LigAB enzyme and a nucleotide sequence of SEQ ID NO: 4 encoding a LigC enzyme, respectively;
(b) obtaining a mixed strain by mixing the obtained first recombinant strain and second recombinant strain at a ratio of 15:25 (cell number); and
(c) producing 2-pyrone-4,6-dicarboxylic acid (PDC) by reacting the obtained mixed strain with terephthalic acid (TPA).

**24.** A method of producing PDC comprising the steps of:

(a) obtaining a recombinant strain introduced with a nucleotide sequence of SEQ ID NO: 3 encoding a LigAB enzyme and a nucleotide sequence of SEQ ID NO: 4 encoding a LigC enzyme; and
(b) producing 2-pyrone-4,6-dicarboxylic acid (PDC) by reacting the obtained recombinant strain with protocatechuic acid (PCA).

**25.** A method of purifying 2-pyrone-4,6-dicarboxylic acid comprising the steps of:

(a) preparing a crude PDC-containing aqueous solution by acidifying crude 2-pyrone-4,6-dicarboxylic acid sodium salt (PDC sodium salt);
(b) extracting the crude PDC-containing aqueous solution resulted in step (a) with an organic solvent to layer-separate a water layer and an organic layer;
(c) separating and removing a white precipitate formed by adding acetone to the water layer resulted in step (b);
(d) extracting the solution from which the white precipitate is removed in step (c) with an organic solvent to be layer-separated into a water layer and an organic layer;
(e) concentrating the organic layer resulted in step (b) and the organic layer resulted in step (d); and
(f) column-chromatographing the concentrate resulted in step (e) with a mixed solvent of ethyl acetate and hexane.

**26.** The method of purifying 2-pyrone-4,6-dicarboxylic acid of claim 25, wherein the PDC sodium salt includes one or more other metal cations selected from the group consisting of monovalent metal cations such as lithium, potassium, rubidium, silver, and cesium; bivalent metal cations such as magnesium, calcium, iron (II), copper (II), zinc, barium, cobalt, nickel (II), manganese, and chromium (II); trivalent metal cations such as iron (III), aluminum, and gallium;

and tetravalent metal cations such as arsenic(IV), lead (IV), titanium (IV), and germanium (IV).

**27.** The method of purifying 2-pyrone-4,6-dicarboxylic acid of claim 26, wherein the acidifying in step (a) is performed using at least one acid selected from the group consisting of hydrochloric acid, sulfuric acid, nitric acid and phosphoric acid.

**28.** The method of purifying 2-pyrone-4,6-dicarboxylic acid of claim 26, wherein the acidifying in step (a) is performed using hydrochloric acid.

**29.** The method of purifying 2-pyrone-4,6-dicarboxylic acid of claim 26, wherein the organic solvents used in steps (b) and (d) are the same as or different from each other, and are at least one or two selected from the group consisting of ethyl acetate, methyl ethyl ketone, THF and mixtures thereof.

**30.** The method of purifying 2-pyrone-4,6-dicarboxylic acid of claim 26, wherein the organic solvents used in steps (b) and (d) are ethyl acetate.

**31.** The method of purifying 2-pyrone-4,6-dicarboxylic acid of claim 26, wherein the column chromatography of step (f) is eluted with a mixed solvent of ethyl acetate:hexane = 4:1 to 1:4.

**32.** The method of purifying 2-pyrone-4,6-dicarboxylic acid of claim 26, wherein the column chromatography of step (f) is gradient-eluted while being changed from the mixed solvent of ethyl acetate:hexane = 4:1 to 1:4 to ethyl acetate (single solvent).

**33.** The method of purifying 2-pyrone-4,6-dicarboxylic acid of claim 26, wherein the column chromatography of step (f) is silica gel column chromatography.

[FIG. 1]

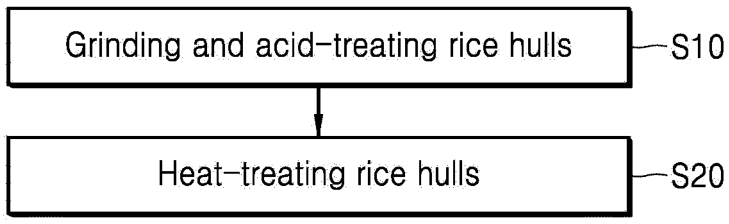
Grinding and acid-treating rice hulls
S10
Heat-treating rice hulls
S20

[FIG. 2]

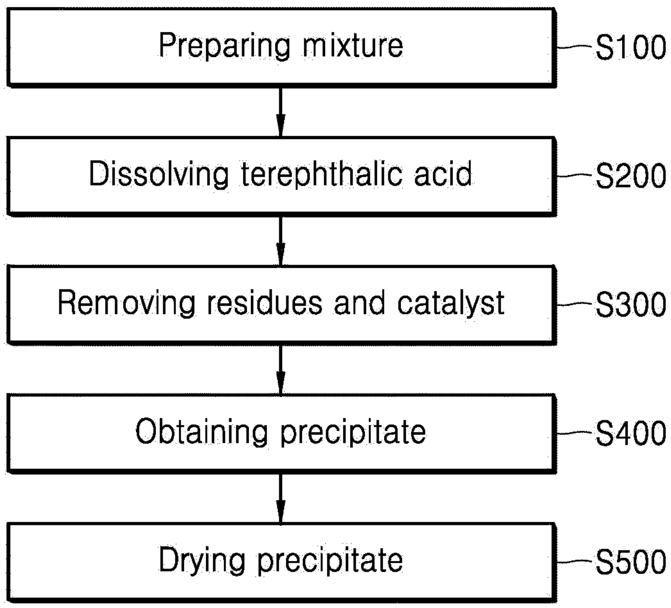
Preparing mixture
S100
Dissolving terephthalic acid
S200
Removing residues and catalyst
S300
Obtaining precipitate
S400
Drying precipitate
S500

[FIG. 3]

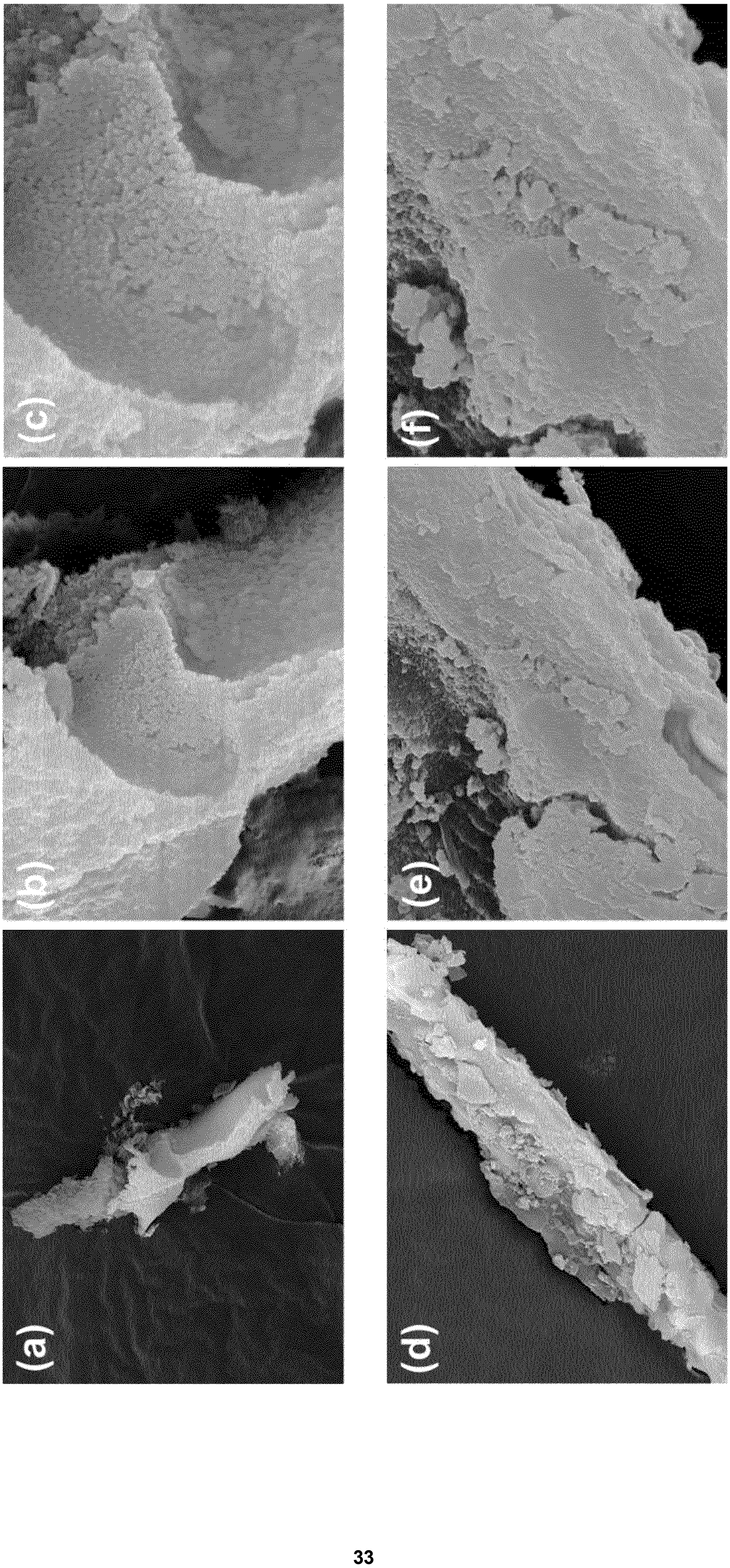

[FIG. 4]

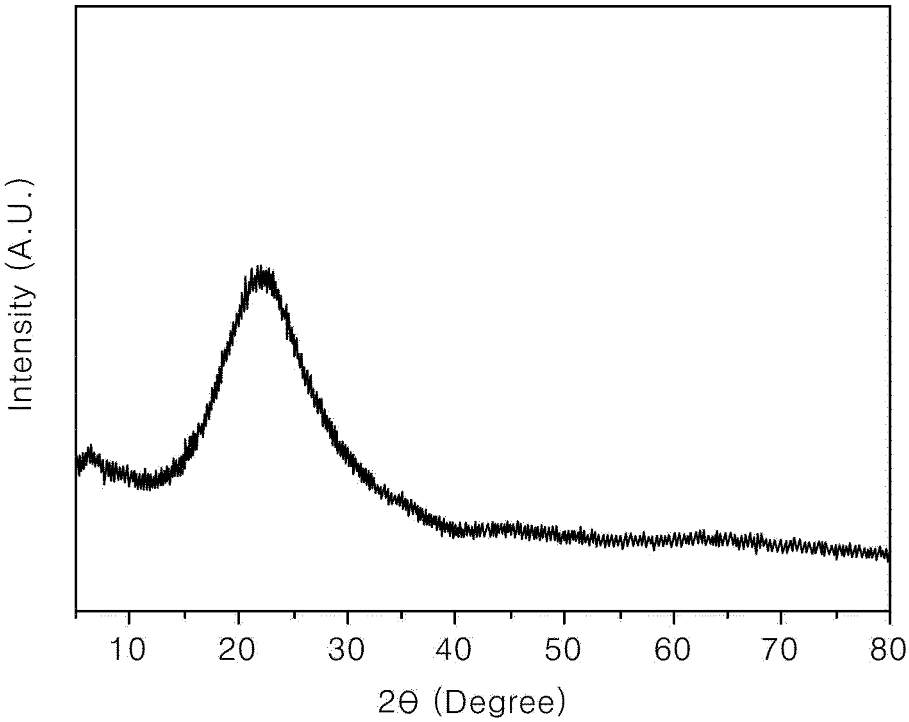

[FIG. 5]

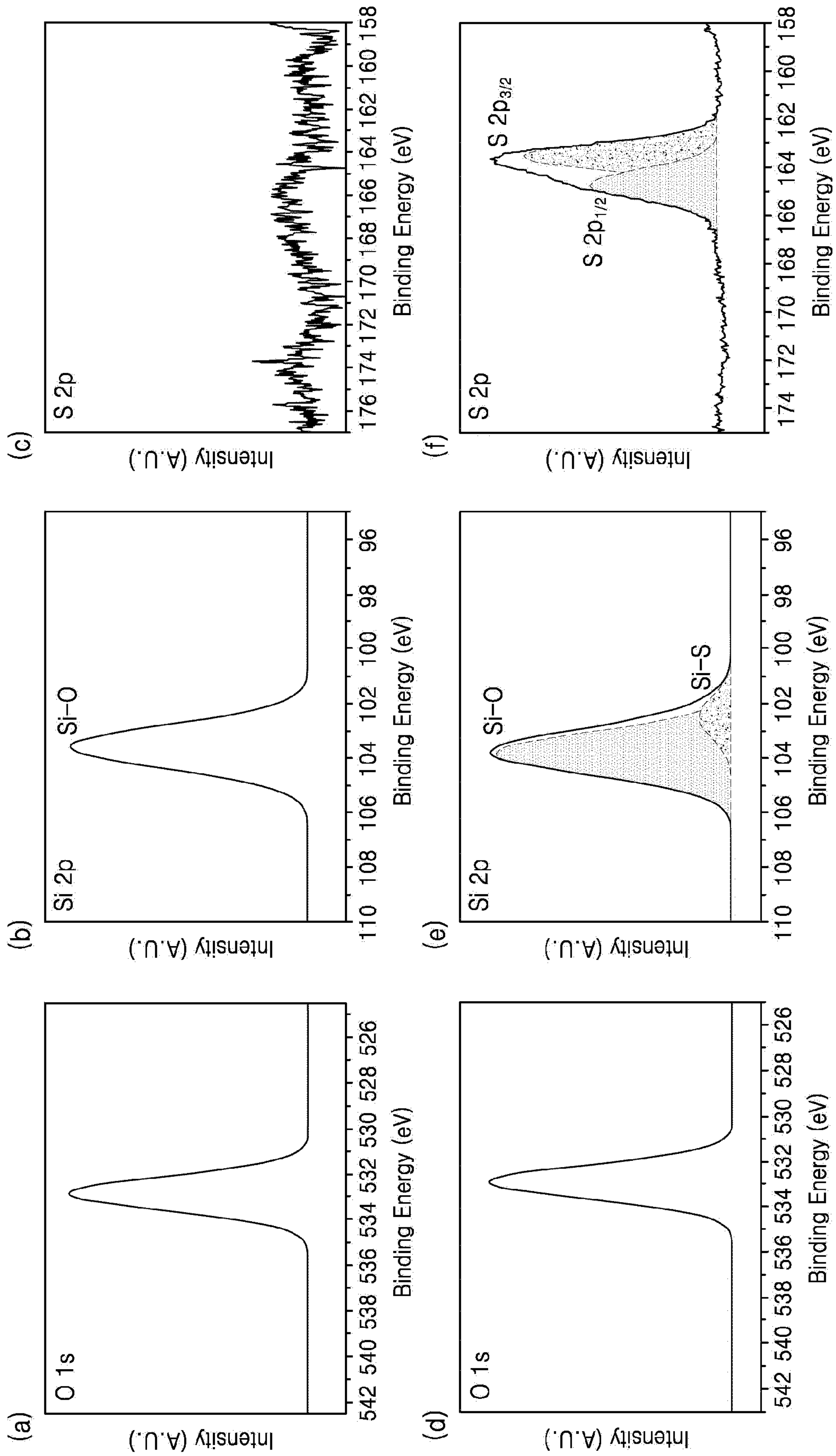
(a)
O 1s
Intensity (A.U.)
Binding Energy (eV)
542 540 538 536 534 532 530 528 526
(b)
Si 2p
Si–O
Intensity (A.U.)
Binding Energy (eV)
110 108 106 104 102 100 98 96
(c)
S 2p
Intensity (A.U.)
Binding Energy (eV)
176 174 172 170 168 166 164 162 160 158
(d)
O 1s
Intensity (A.U.)
Binding Energy (eV)
542 540 538 536 534 532 530 528 526
(e)
Si 2p
Si–O
Si–S
Intensity (A.U.)
Binding Energy (eV)
110 108 106 104 102 100 98 96
(f)
S 2p
S 2p1/2
S 2p3/2
Intensity (A.U.)
Binding Energy (eV)
174 172 170 168 166 164 162 160 158

[FIG. 6]

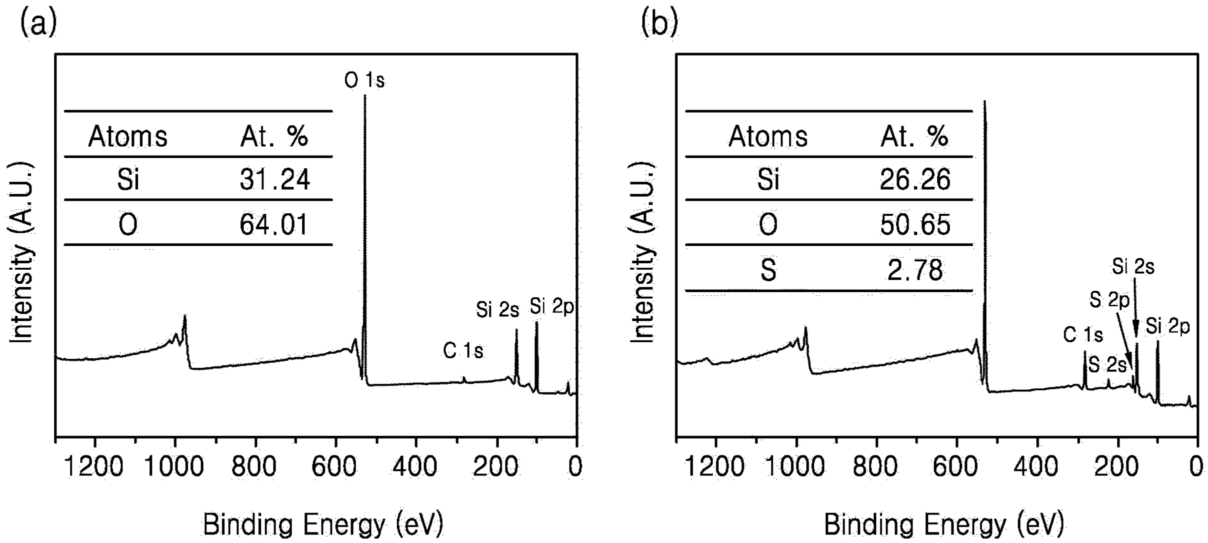
(a)
Atoms At. %
Si 31.24
O 64.01
O 1s
Si 2s
Si 2p
C 1s
Intensity (A.U.)
1200 1000 800 600 400 200 0
Binding Energy (eV)
(b)
Atoms At. %
Si 26.26
O 50.65
S 2.78
Si 2s
S 2p
C 1s
S 2s
Si 2p
Intensity (A.U.)
1200 1000 800 600 400 200 0
Binding Energy (eV)

[FIG. 7]

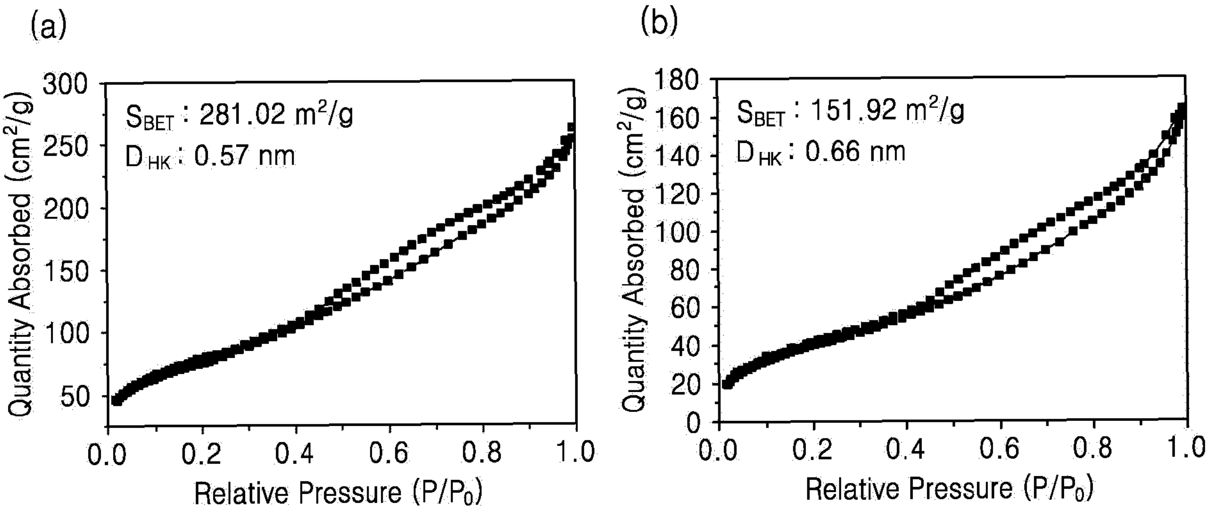
(a)
(b)
$S_{BET}$ : 281.02 $m^2/g$
$D_{HK}$ : 0.57 nm
$S_{BET}$ : 151.92 $m^2/g$
$D_{HK}$ : 0.66 nm
Quantity Absorbed ($cm^2/g$)
Relative Pressure ($P/P_0$)
300
250
200
150
100
50
180
160
140
120
100
80
60
40
20
0
0.0
0.2
0.4
0.6
0.8
1.0

[FIG. 8]

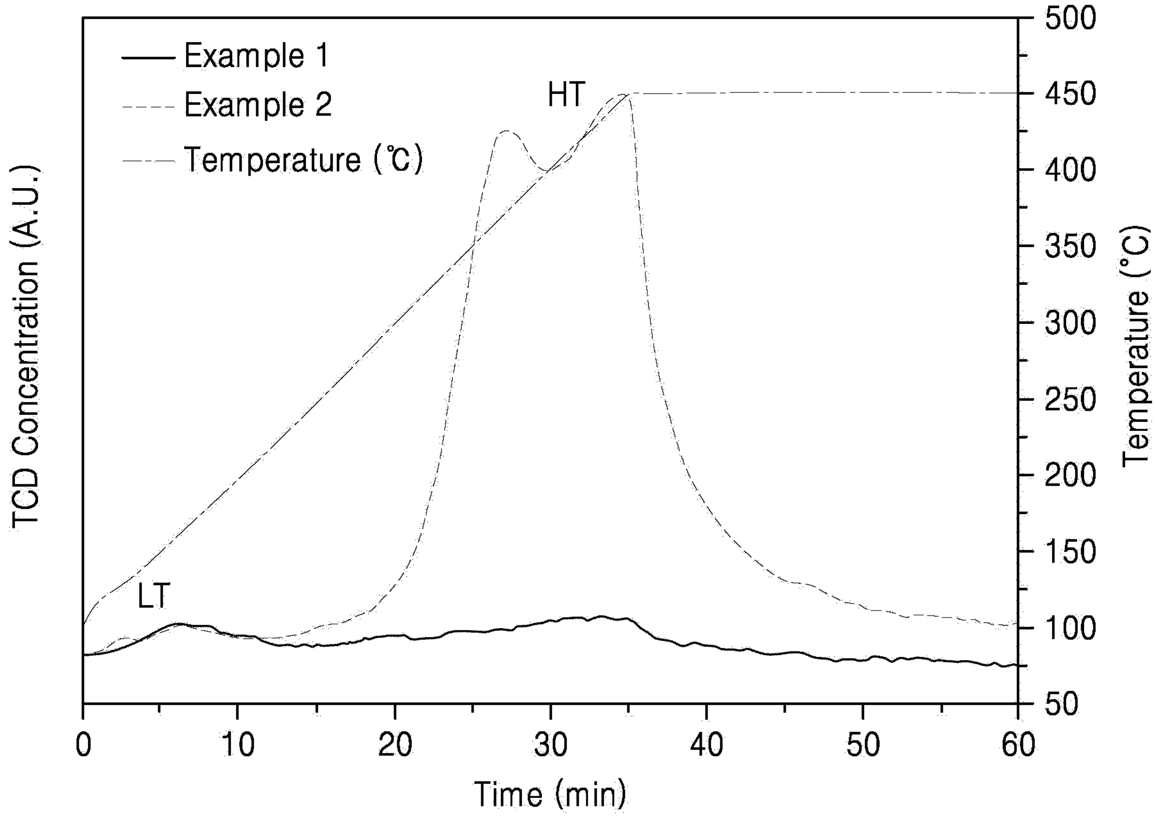


| | Example 1 | Example 2 |
|---|---|---|
| LT peak (°C) | 161.6 | 161.6 |
| Acidic sites (mmol/g) | 0.00838 | 0.00838 |
| HT peak (°C) | – | 369.4 |
| Acidic sites (mmol/g) | – | 0.39319 |

[FIG. 9]

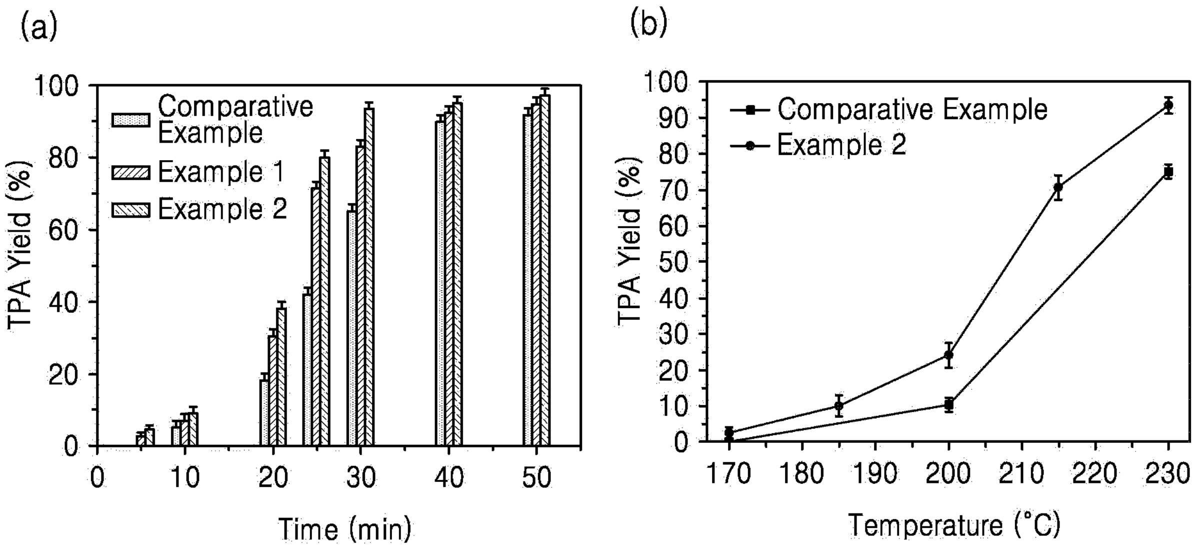
(a)
(b)
TPA Yield (%)
Comparative Example
Example 1
Example 2
Time (min)
Temperature (°C)
0
10
20
30
40
50
60
70
80
90
100
170
180
190
200
210
220
230

[FIG. 10]

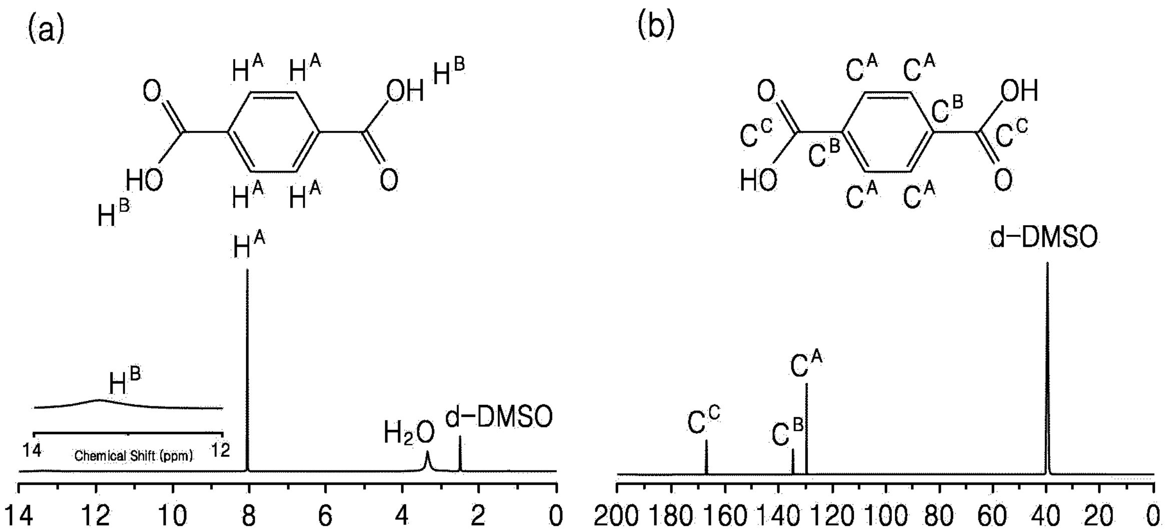
(a)
(b)
$H^A$
$H^B$
14 Chemical Shift (ppm) 12
$H_2O$
d-DMSO
14 12 10 8 6 4 2 0
d-DMSO
$C^A$
$C^C$
$C^B$
200 180 160 140 120 100 80 60 40 20 0

[FIG. 11]

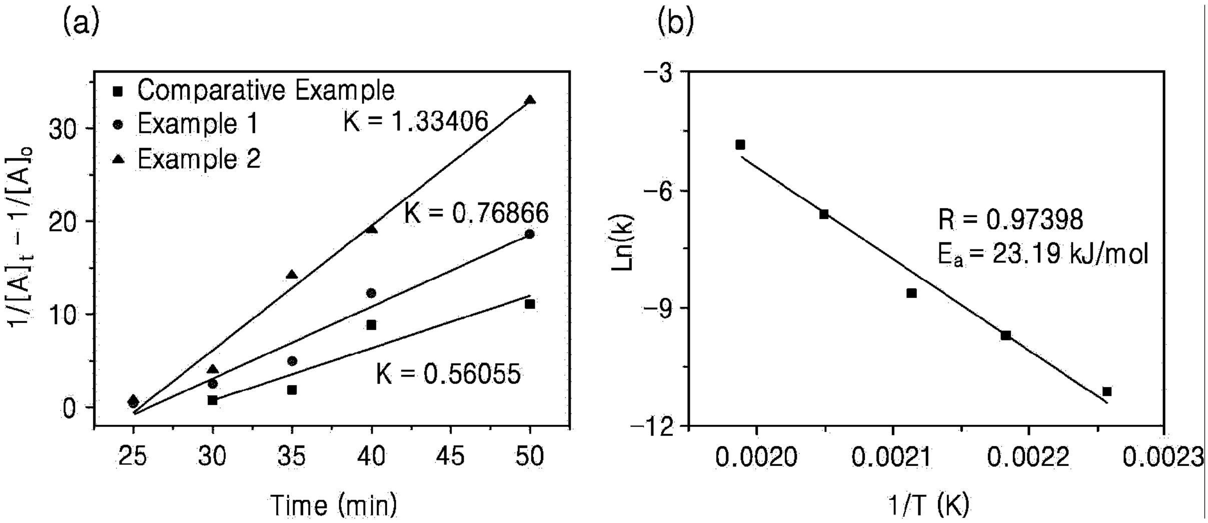
(a)
Comparative Example
Example 1
Example 2
K = 1.33406
K = 0.76866
K = 0.56055
1/[A]t - 1/[A]o
Time (min)
0
10
20
30
25
30
35
40
45
50
(b)
R = 0.97398
Ea = 23.19 kJ/mol
Ln(k)
1/T (K)
-3
-6
-9
-12
0.0020
0.0021
0.0022
0.0023

[FIG. 12]

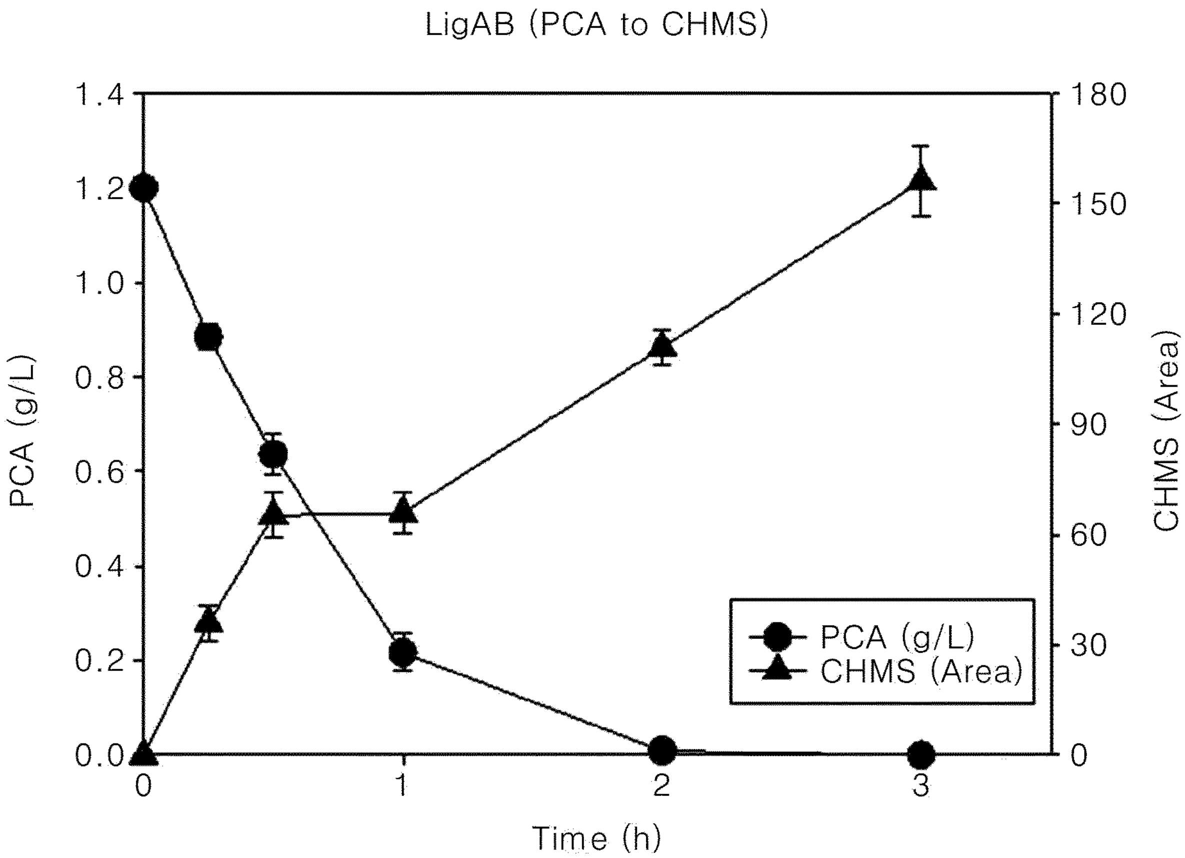
LigAB (PCA to CHMS)
PCA (g/L)
CHMS (Area)
Time (h)
1.4
1.2
1.0
0.8
0.6
0.4
0.2
0.0
180
150
120
90
60
30
0
0
1
2
3
PCA (g/L)
CHMS (Area)

[FIG. 13]

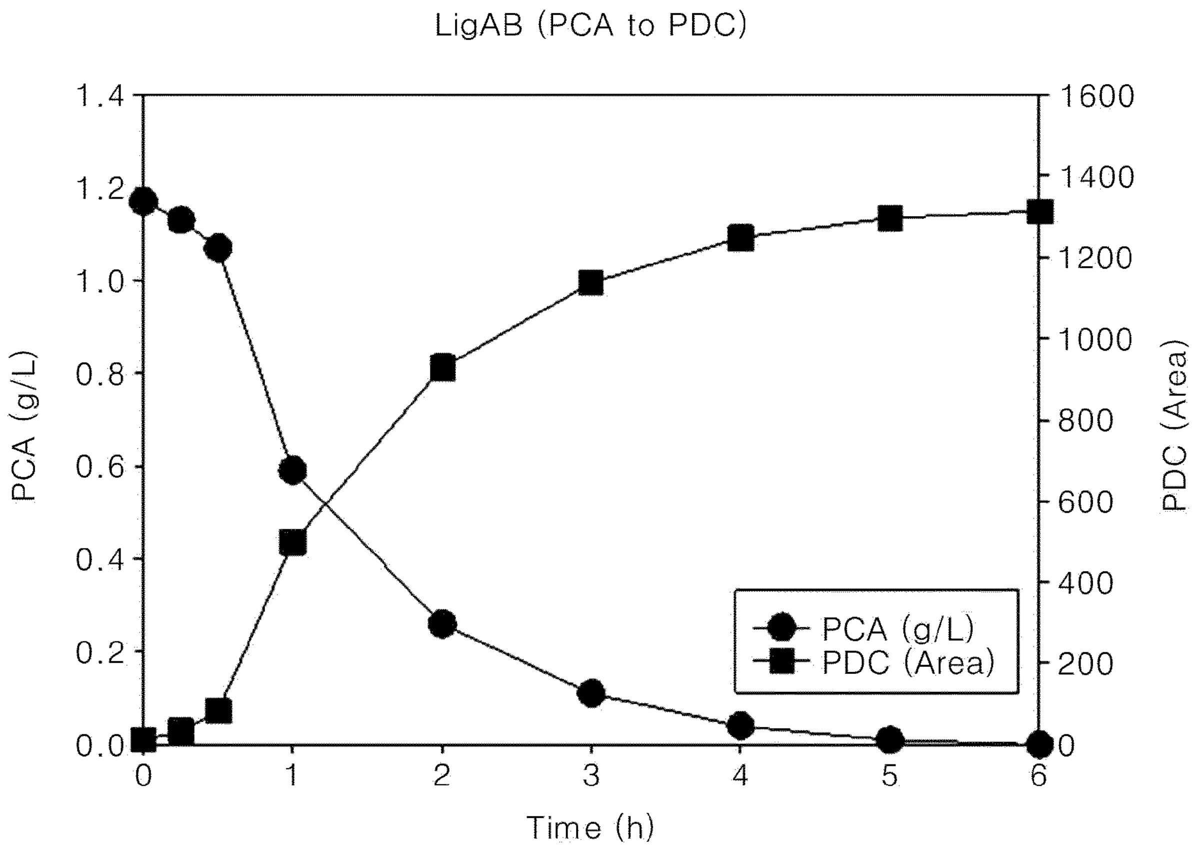
LigAB (PCA to PDC)
PCA (g/L)
PDC (Area)
Time (h)
0.0
0.2
0.4
0.6
0.8
1.0
1.2
1.4
0
200
400
600
800
1000
1200
1400
1600
0
1
2
3
4
5
6
PCA (g/L)
PDC (Area)

[FIG. 14]

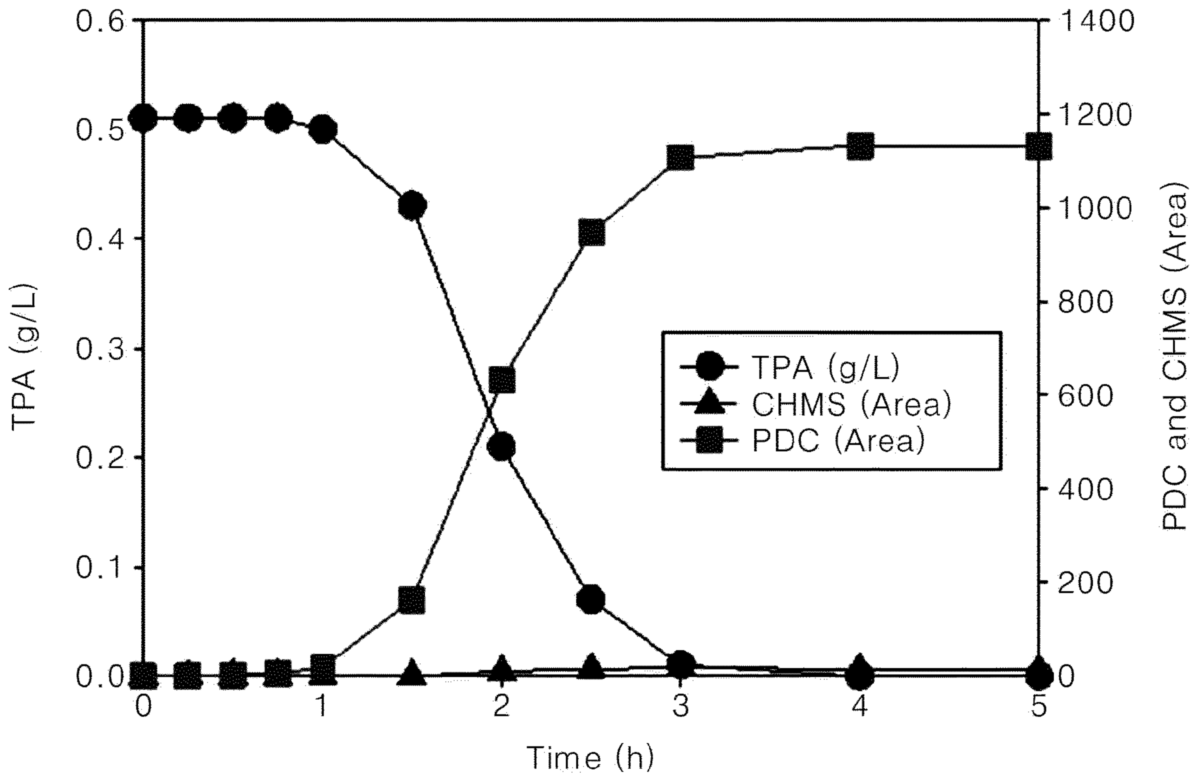
0.6
0.5
0.4
0.3
0.2
0.1
0.0
TPA (g/L)
1400
1200
1000
800
600
400
200
0
PDC and CHMS (Area)
0
1
2
3
4
5
Time (h)
TPA (g/L)
CHMS (Area)
PDC (Area)

[FIG. 15]

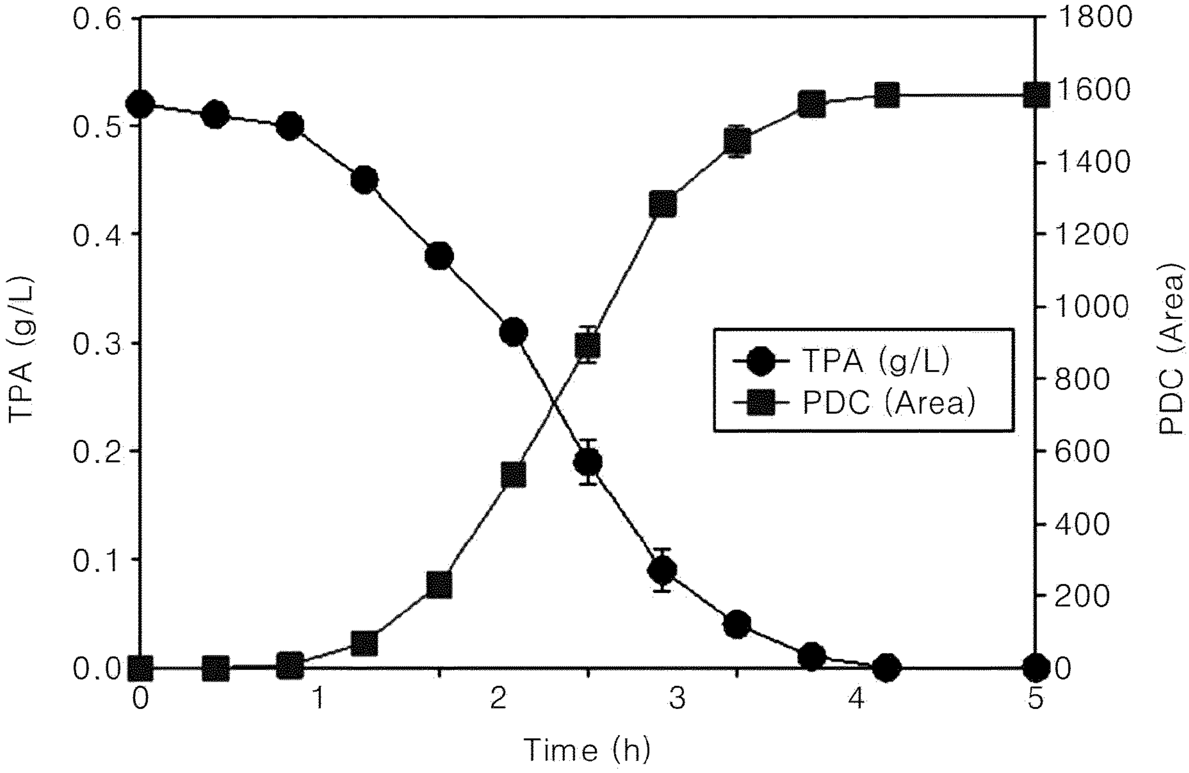
0.6
0.5
0.4
0.3
0.2
0.1
0.0
TPA (g/L)
1800
1600
1400
1200
1000
800
600
400
200
0
PDC (Area)
0
1
2
3
4
5
Time (h)
TPA (g/L)
PDC (Area)

[FIG. 16]

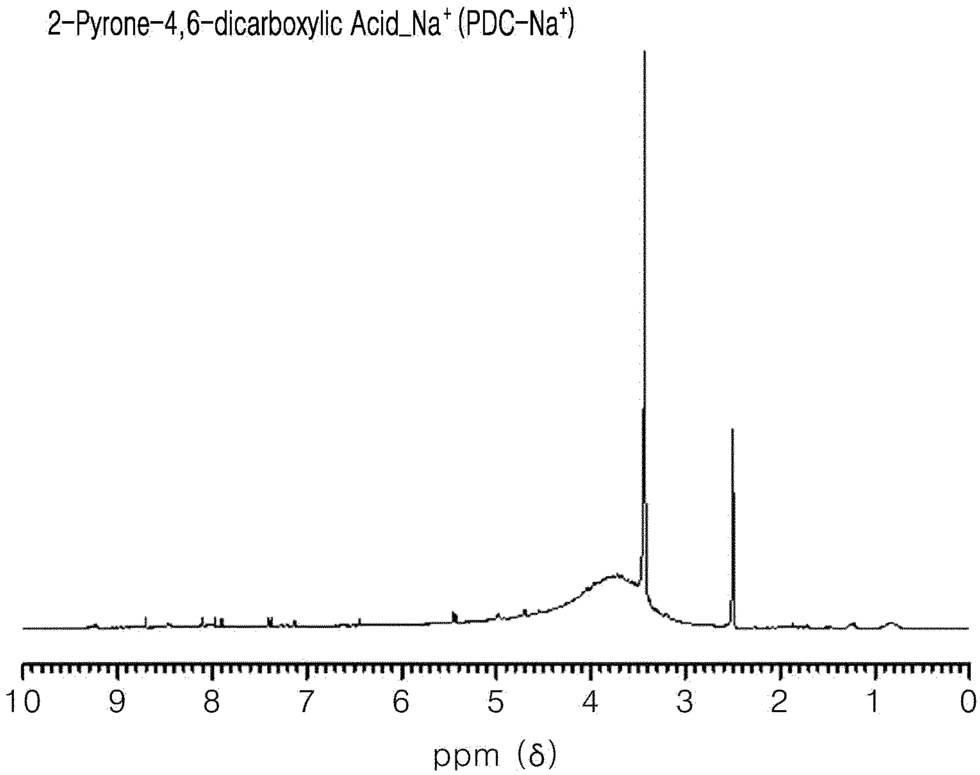

[FIG. 17]

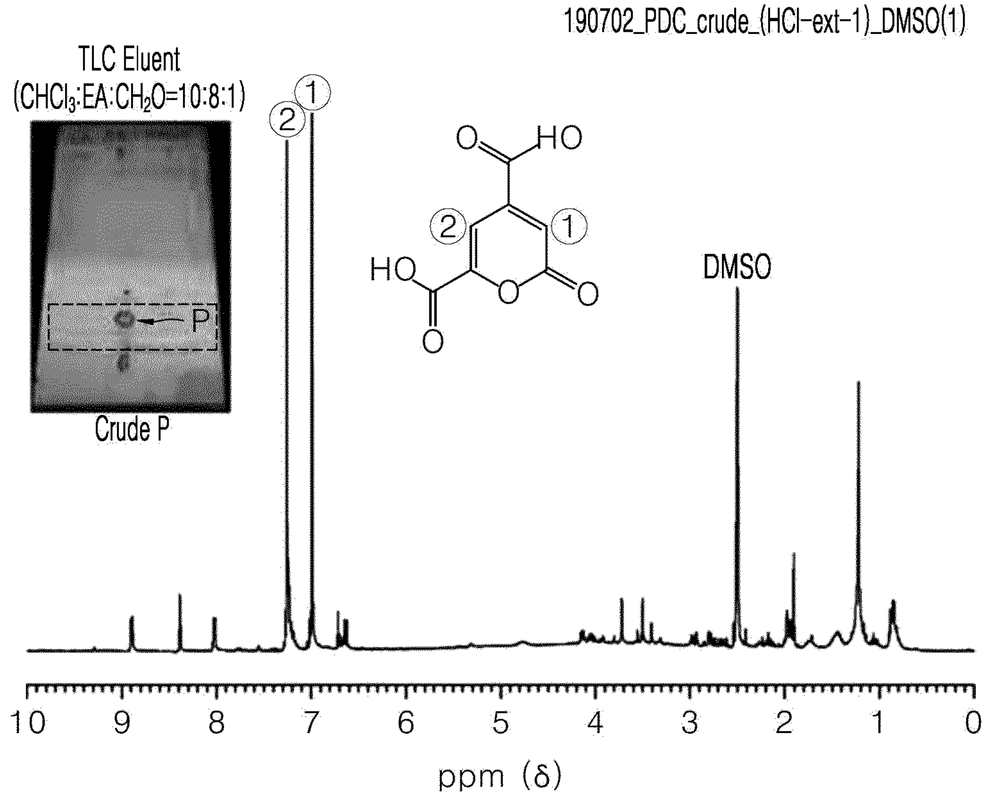
190702_PDC_crude_(HCl-ext-1)_DMSO(1)
TLC Eluent
(CHCl3:EA:CH2O=10:8:1)
P
Crude P
DMSO
10 9 8 7 6 5 4 3 2 1 0
ppm (δ)

[FIG. 18]

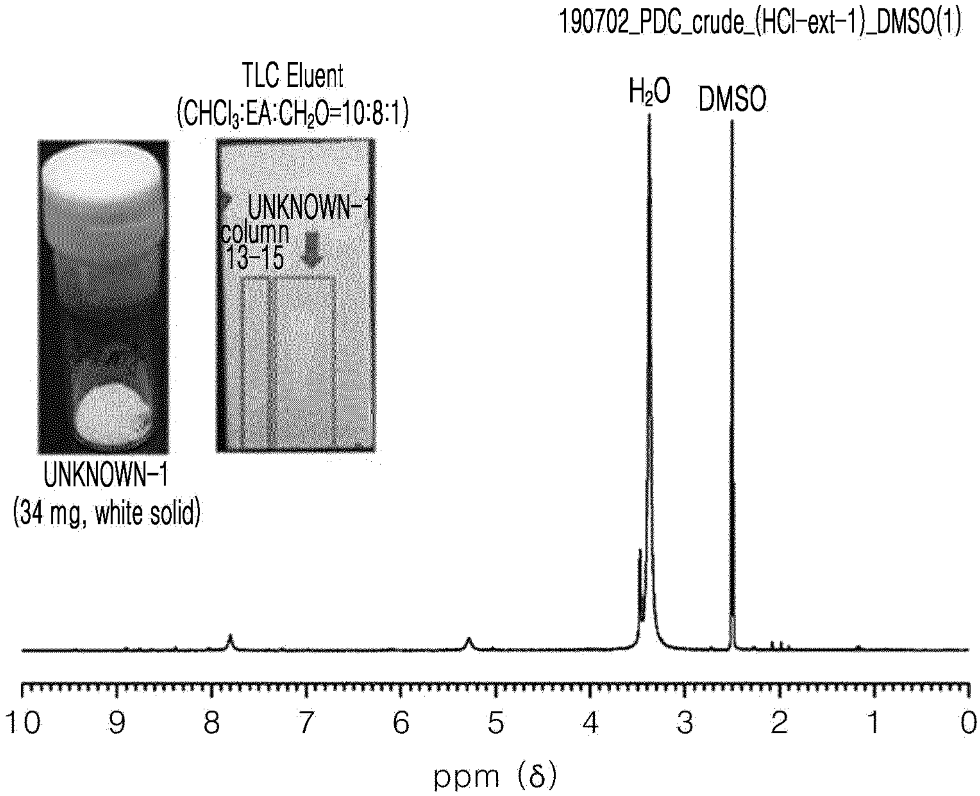
190702_PDC_crude_(HCl-ext-1)_DMSO(1)
TLC Eluent
(CHCl3:EA:CH2O=10:8:1)
UNKNOWN-1
column
13-15
UNKNOWN-1
(34 mg, white solid)
H2O
DMSO
10
9
8
7
6
5
4
3
2
1
0
ppm (δ)

[FIG. 19]

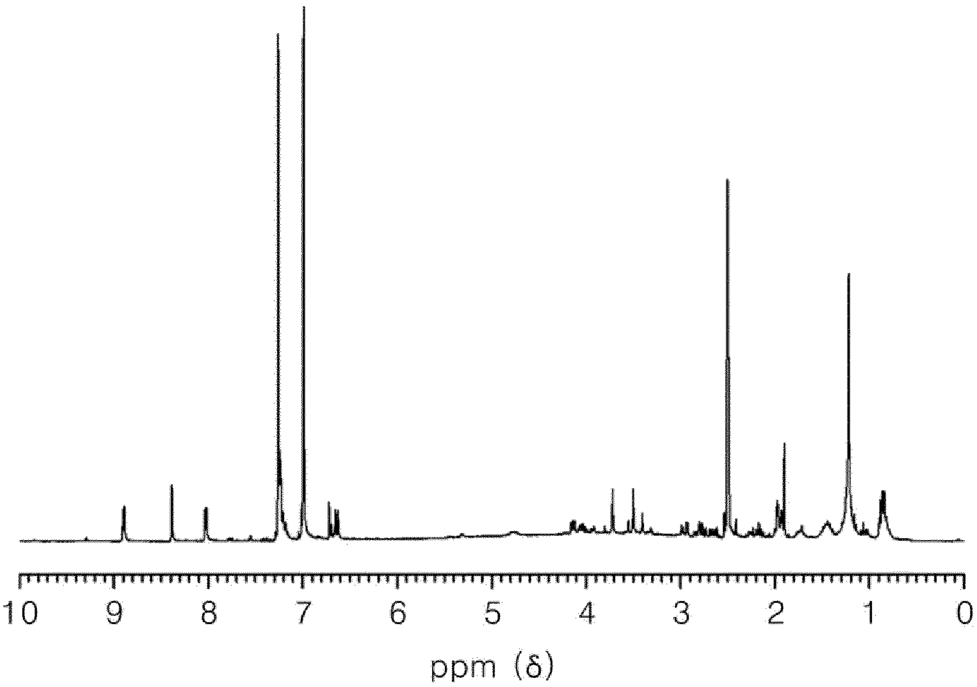

[FIG. 20]

Column(2)-PDC product(EA : MeOH = 2 : 1)

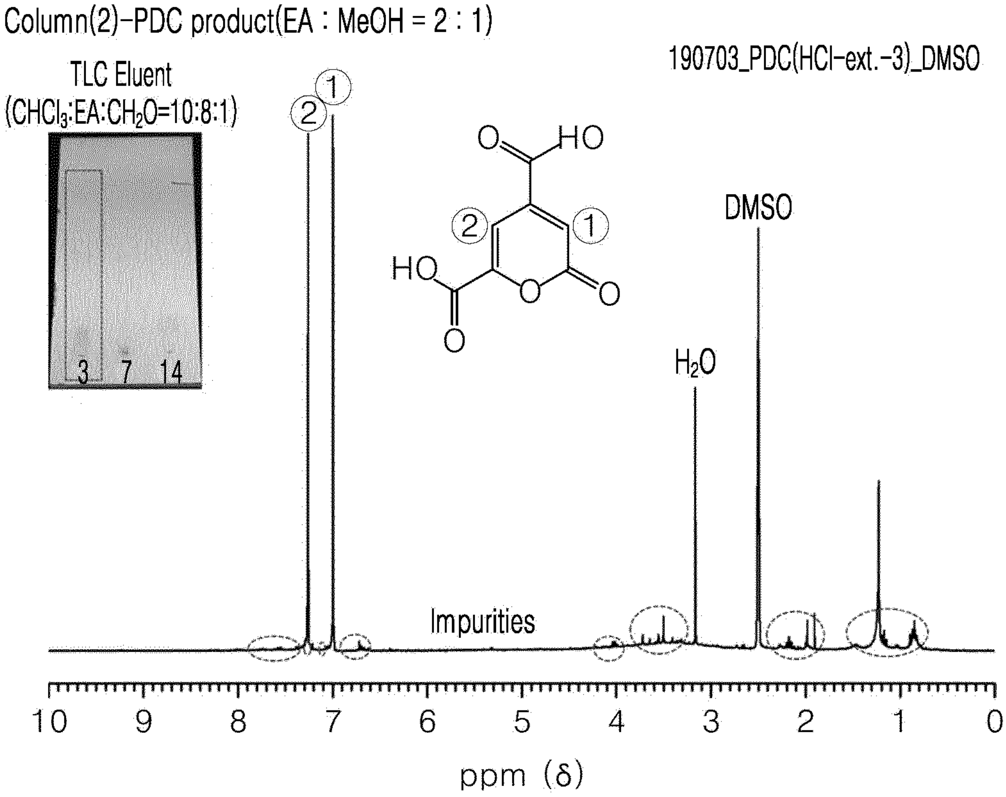

[FIG. 21]

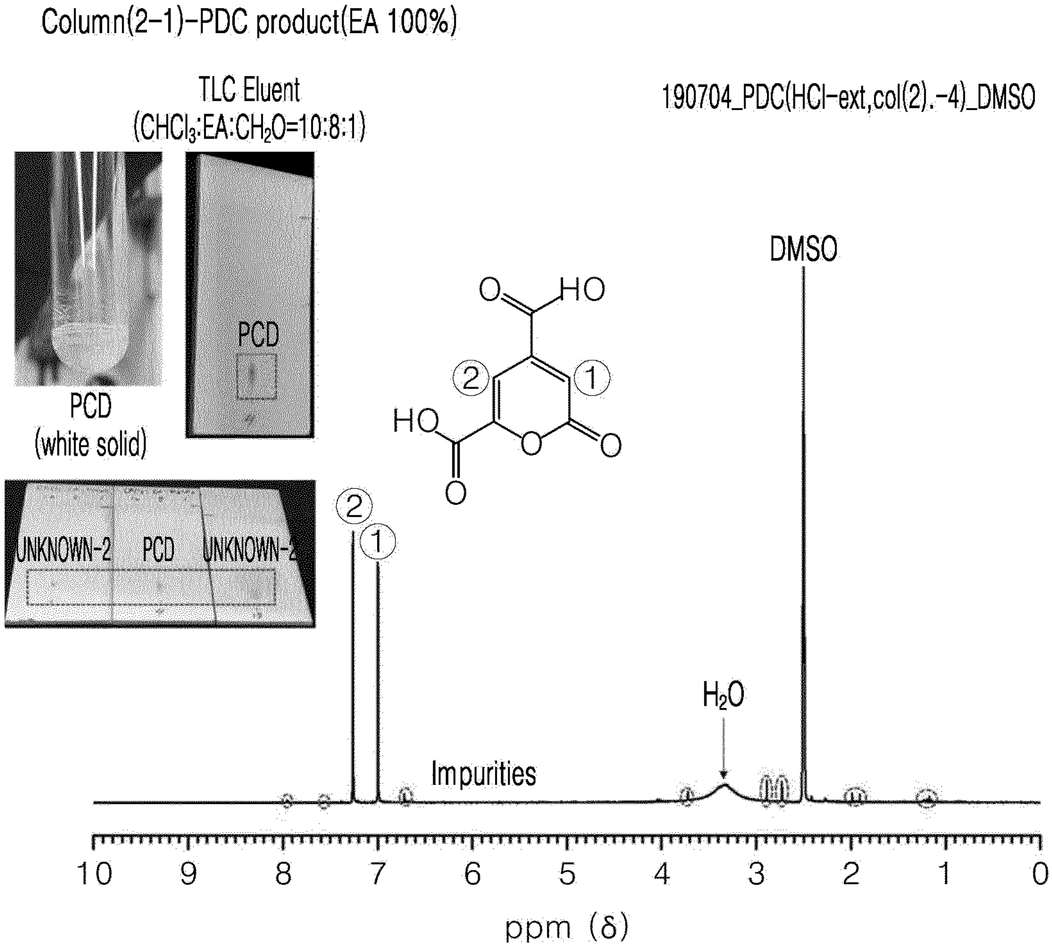
Column(2-1)-PDC product(EA 100%)
TLC Eluent
(CHCl3:EA:CH2O=10:8:1)
PCD
PCD
(white solid)
UNKNOWN-2
PCD
UNKNOWN-2
190704_PDC(HCl-ext,col(2).-4)_DMSO
DMSO
H2O
Impurities
10
9
8
7
6
5
4
3
2
1
0
ppm (δ)

[FIG. 22]

Column(3)-PDC product(EA : Hex = 1 : 1)